# EUROPEAN PATENT APPLICATION

(11) **EP 4 506 358 A1**
(43) Date of publication of application: **12.02.2025**
(21) Application number: 23799588.1
(22) Date of filing: 12.04.2023
(51) Int. Cl.: C07K 14/47, A61P 35/00, A61K 38/00, G01N 33/574

(54) **ICOS-L VARIANT WITH ENHANCED BINDING AFFINITY FOR ICOS**

(30) Priority: 03.05.2022 KR 20220054766
(71) Applicant: Korea University Research and Business Foundation, Seoul 02841 (KR)
(72) Inventor: JUNG, Sang Taek, Seoul 06217 (KR); HA, Ji Yeon, Seoul 05401 (KR)
(74) Representative: Isarpatent
(86) International application number: PCT/KR2023/004936
(87) International publication number: WO 2023/214705

(57) **Abstract**

The present disclosure relates to ICOS-L variants with enhanced binding affinity for ICOS. The ICOS-L variants of the present disclosure have significantly enhanced binding affinity for ICOS (~approximately 100 times) compared to the wild-type ICOS-L and previous ICOS-L variants. With sizes considerably smaller than the large molecule IgG antibody, the variants easily penetrate the tumor microenvironment. It is straightforward to produce the variants, and their fusion with various immunotherapies makes applications as therapeutic agents and imaging molecules feasible. Therefore, the variants can be effectively utilized for cancer treatment and diagnosis.

## Description

### [Technical Field]

The present disclosure relates to ICOS-L variants with enhanced binding affinity for ICOS.

### [Background Art]

Medicines for cancer treatment are largely divided into low molecular weight medicines and high molecular weight medicines, and the high molecular weight medicines with specificity are in the spotlight as therapeutic agents compared to the low molecular weight medicines with relatively large side effects without specificity. Cancer cells express, on a cell surface, an immune checkpoint protein, which is used when normal cells suppress immune cell activation in order to evade a killing mechanism by immune cells, and recently, research on an immune checkpoint inhibitory protein has been actively conducted as a method for treating cancer. While it has been reported that patients who respond to immune checkpoint inhibitors have fewer side effects during treatment, but have superior therapeutic effects to conventional anticancer drugs for various carcinomas, the use thereof is rapidly increasing. However, more than half of patients do not still respond to clinically approved immune checkpoint inhibitors, and some early responders are observed to have resistance to cancer developing again after treatment. Since the causes of resistance to the immune checkpoint inhibitors vary depending on a tumor immunological characteristic of each patient, new biomarkers capable of selecting patients suitable for treatment are required for effective treatment and reduction of medical costs. Many preclinical and clinical studies have reported that combined therapy using immunostimulatory drugs improves patient response rates, and thus effective immunostimulatory therapeutic agents are also urgently needed.

Recently, various antibody therapeutic agents have been developed to inhibit the action of immune checkpoint proteins such as Cytotoxic T-Lymphocyte-Associated Protein 4 (CTLA-4) and Programmed Cell Death Protein 1 (PD-1), and then clinical demand is explosively increasing. Accordingly, the immune checkpoint inhibitor market has been expected to grow steadily at an annual average rate of 21.8% from 2020 to 2027. However, since the antibody is a macromolecular protein having a molecular weight of 150,000, it is difficult to penetrate into cancer tissue, and thus, there is a disadvantage that it is difficult to inhibit the immune checkpoint proteins of tumor cells and immune cells in a tumor micro-environment. For more effective treatment, there is a need for a protein therapeutic agent that is much smaller than the size of the antibody and easily penetrates into the cancer tissue.

Meanwhile, an inducible co-stimulator (ICOS), which is a co-stimulatory receptor for T cell activation, is a T cell-specific co-stimulatory molecule that activates T cell responses to external antigens, and as it has been reported that the ICOS is related to the treatment prognosis of various carcinomas, the ICOS is also being suggested to have a potential as a biomarker for predicting treatment responses to immune checkpoint inhibitors as well as therapeutic agents for immune stimulation. To observe T cells present in the tumor micro-environment, verification of ICOS expression is required through real-time imaging using radioactive isotopes or staining after separating cancer tissue, and it is preferred to use small protein molecules that are much smaller than large molecules such as antibodies and may easily penetrate into cancer tissue. In addition, ICOS target substances have also been verified in the effect as combined therapeutic agents with immune checkpoint inhibitors, and thus researches have been conducted to develop therapeutic agents in two aspects of further activating cytotoxic T cells, which are very important for killing cancer cells, by promoting the activity of ICOS, or inducing the anticancer efficacy by killing regulatory T cells that express ICOS. However, wild-type ICOS-L has very low affinity for ICOS (equilibrium dissociation constant = ~ 700 nM), and thus has a problem that it is difficult to be used.

### [Disclosure]

### [Technical Problem]

An object of the present disclosure is to provide an ICOS-L variant.

Another object of the present disclosure is to provide an immunomodulatory protein comprising the ICOS-L variant.

Yet another object of the present disclosure is to provide a conjugate comprising the ICOS-L variant.

Yet another object of the present disclosure is to provide a T-cell activator.

Yet another object of the present disclosure is to provide an engineered cell.

Yet another object of the present disclosure is to provide an anticancer adjuvant.

Still another object of the present disclosure is to provide a pharmaceutical composition for treating or preventing cancer.

Still another object of the present disclosure is to provide a composition for diagnosing cancer.

Still another object of the present disclosure is to provide a method for providing information for diagnosing cancer.

Still another object of the present disclosure is to provide a method for predicting therapeutic responses or diagnosing prognosis for an immune checkpoint inhibitor.

Still another object of the present disclosure is to provide a use of an ICOS-L variant, an immunomodulatory protein, a conjugate or an engineered cell for preventing or treating cancer.

Still another object of the present disclosure is to provide a method for treating cancer.

### [Technical Solution]

In order to achieve the aspects, the present disclosure provides an ICOS-L variant with enhanced binding affinity for ICOS.

Further, the present disclosure provides an immunomodulatory protein including the ICOS-L variant and an Fc domain of immunoglobulin or a variant thereof.

Further, the present disclosure provides a conjugate including the ICOS-L variant or the immunomodulatory protein and a targeting moiety.

Further, the present disclosure provides a T-cell activator including the ICOS-L variant, the immunomodulatory protein or the conjugate.

Further, the present disclosure provides an engineering cell including the ICOS-L variant, the immunomodulatory protein or the conjugate.

Further, the present disclosure provides an anticancer adjuvant including the ICOS-L variant, the immunomodulatory protein, the conjugate or the engineering cell.

Further, the present disclosure provides a pharmaceutical composition for treating or preventing cancer including the ICOS-L variant, the immunomodulatory protein, the conjugate or the engineering cell.

Further, the present disclosure provides a composition for diagnosing cancer including the ICOS-L variant, the immunomodulatory protein, the conjugate or the engineering cell.

Further, the present disclosure provides a method for providing information for diagnosing cancer.

Further, the present disclosure provides a method for predicting therapeutic responses or diagnosing prognosis for an immune checkpoint inhibitor.

Further, the present disclosure provides a use of an ICOS-L variant, an immunomodulatory protein, a conjugate or an engineered cell for preventing or treating cancer.

Further, the present disclosure provides a method for treating cancer including administering an ICOS-L variant, an immunomodulatory protein, a conjugate or an engineered cell in a pharmaceutically effective amount to a subject suffering with cancer.

### [Advantageous Effects]

According to the present disclosure, the ICOS-L variants have significantly enhanced binding affinity for ICOS (~approximately 100 times) compared to the wild-type ICOS-L and previous ICOS-L variants. With sizes considerably smaller than the large molecule IgG antibody, the variants easily penetrate the tumor microenvironment. It is straightforward to produce the variants, and their fusion with various immunotherapies makes applications as therapeutic agents and imaging molecules feasible. Therefore, the variants can be effectively utilized for cancer treatment and diagnosis.

### [Description of Drawings]

FIG. 1 is a diagram showing an SDS-PAGE gel photograph of an expression vector for an ICOS-Fc protein and a purified protein.
FIG. 2 is a diagram showing binding affinity for purified ICOS-Fc and wild-type ICOS-L analyzed by ELISA:
   PD-L1 WT: Negative control.
FIG. 3 is a diagram illustrating a result of flow cytometry for selecting an ICOS-L display method.
FIG. 4 is a diagram illustrating a result of amino acid sequencing of a constructed initial library.
FIG. 5 is a diagram illustrating a result of verifying library amplification by a flow cytometer according to a screening process.
FIG. 6 is a diagram illustrating a result of analyzing binding affinity ICOS-L variants for ICOS using a flow cytometer.
FIG. 7 is a diagram illustrating SDS-PAGE gel photographs of wild-type ICOS-L, control A184, and six ICOS-L variants Y3, Y8, Y13, Y20, Y35, and Y48 discovered in the present disclosure that are expressed and purified.
FIG. 8 is a diagram of analyzing ICOS binding affinity of wild-type ICOS-L, control A184, and six ICOS-L variants discovered in the present disclosure by ELISA.
FIG. 9 is a diagram illustrating SDS-PAGE gel photographs of expressed and purified ICOS-L variants Y8_P51Q and Y8_H57N for critical mutational analysis of a Y8 variant.
FIG. 10 is a diagram of analyzing binding affinity of wild-type ICOS-L, control A184, and variants Y8, Y8_P51Q and Y8_H57N for ICOS by ELISA.
FIG. 11 shows sensor grams of wild-type ICOS-L, control A184, and variants Y8, Y8_P51Q and Y8_H57N measured using Octet.
FIG. 12 illustrates KD values of wild-type ICOS-L, control A184, and variants Y8, Y8_P51Q and Y8_H57N measured using Octet.
FIG. 13 is a diagram showing an SDS-PAGE gel photograph of a purified ICOS monomer protein.
FIG. 14 is a diagram showing SDS-PAGE gel photographs of wild-type ICOS-L, A184, and variants Y8 and Y8_H57N of the present disclosure, which are fused to expressed and purified Fc.
FIG. 15 is a diagram illustrating binding affinity of expressed and purified ICOS-L-Fcs (wild-type-Fc, A184-Fc, Y8-Fc, and Y8_H57N-Fc) and wild-type ICOS analyzed by ELISA.
FIG. 16 is a diagram showing results of verifying the degree of T cell differentiation induced by PD-L1-Fc and ICOS-L-Fcs (wild-type-Fc, A184-Fc, Y8-Fc, and Y8_H57N-Fc) by a flow cytometer.
FIG. 17 is a diagram showing results of verifying interferon gamma secretion amounts of T cells induced by PD-L1-Fc and ICOS-L-Fcs (wild-type-Fc, A184-Fc, Y8-Fc, and Y8_H57N-Fc) by ELISA.
FIG. 18 is a diagram showing a SDS-PAGE gel photograph of Atezolizumab (ATZ) with a LALAPG mutation introduced into an Fc domain.
FIG. 19 is a diagram showing SDS-PAGE gel photographs of proteins (ICOS-L fused to an antibody) in which a heavy chain of Atezolizumab with the LALAPG mutation introduced into the Fc domain is fused with wild-type ICOS-L, ICOS-LA184 variant or ICOS-L variants Y8 and Y8_H57N of the present disclosure.
FIG. 20 is a diagram showing SDS-PAGE gel photographs of proteins (ICOS-L fused to an antibody) in which a light chain of Atezolizumab with the LALAPG mutation introduced into the Fc domain is fused with wild-type ICOS-L, ICOS-LA184 variant or ICOS-L variants Y8 and Y8_H57N of the present disclosure.
FIG. 21 is a diagram of analyzing binding affinity of wild-type ICOS for proteins in which the heavy chain of Atezolizumab is fused with wild-type ICOS-L, ICOS-L A184 variant or ICOS-L variants Y8 and Y8_H57N of the present disclosure:
   ATZ: Atezolizumab with LALAPG mutation introduced into Fc domain;
   ATZ(H)-Wild-type: Protein in which the wild-type ICOS-L is fused to the heavy chain of Atezolizumab with LALAPG mutation introduced into the Fc domain;
   ATZ(H)-A184: Protein in which the ICOS-LA184 variant is fused to the heavy chain of Atezolizumab with LALAPG mutation introduced into the Fc domain;
   ATZ(H)-Y8: Protein in which the ICOS-L Y8 variant is fused to the heavy chain of Atezolizumab with LALAPG mutation introduced into the Fc domain; and
   ATZ(H)-Y8_H57N: Protein in which the ICOS-L Y8_H57N variant is fused to the heavy chain of Atezolizumab with LALAPG mutation introduced into the Fc domain.

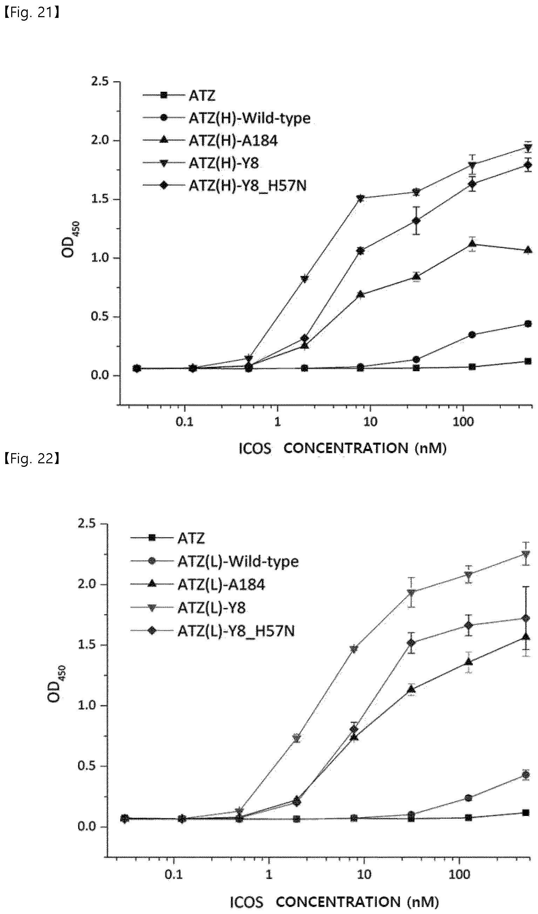

FIG. 22 is a diagram of analyzing binding affinity of wild-type ICOS for proteins in which the light chain of Atezolizumab is fused with wild-type ICOS-L, ICOS-L A184 variant or ICOS-L variants Y8 and Y8_H57N of the present disclosure:
   ATZ: Atezolizumab with LALAPG mutation introduced into an Fc domain;
   ATZ(L)-Wild-type: Protein in which the wild-type ICOS-L is fused to the light chain of Atezolizumab with LALAPG mutation introduced into the Fc domain;
   ATZ(L)-A184: Protein in which the ICOS-L A184 variant is fused to the light chain of Atezolizumab with LALAPG mutation introduced into the Fc domain;
   ATZ(L)-Y8: Protein in which the ICOS-L Y8 variant is fused to the light chain of Atezolizumab with LALAPG mutation introduced into the Fc domain; and
   ATZ(L)-Y8_H57N: Protein in which the ICOS-L Y8_H57N variant is fused to the light chain of Atezolizumab with LALAPG mutation introduced into the Fc domain.

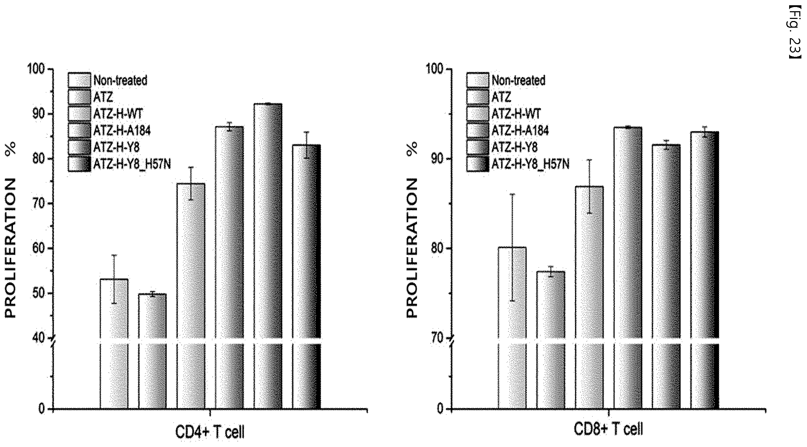

FIG. 23 is a diagram illustrating results of verifying the degree of T cell differentiation induced by proteins (50 nM) in which the heavy chain of Atezolizumab is fused with wild-type ICOS-L, ICOS-L A184 variant, or ICOS-L variants Y8 and Y8_H57N of the present disclosure by a flow cytometer:
   ATZ: Atezolizumab with LALAPG mutation introduced into an Fc domain;
   ATZ(H)-Wild-type: Protein in which the wild-type ICOS-L is fused to the heavy chain of Atezolizumab with LALAPG mutation introduced into the Fc domain;
   ATZ(H)-A184: Protein in which the ICOS-LA184 variant is fused to the heavy chain of Atezolizumab with LALAPG mutation introduced into the Fc domain;
   ATZ(H)-Y8: Protein in which the ICOS-L Y8 variant is fused to the heavy chain of Atezolizumab with LALAPG mutation introduced into the Fc domain; and
   ATZ(H)-Y8_H57N: Protein in which the ICOS-L Y8_H57N variant is fused to the heavy chain of Atezolizumab with LALAPG mutation introduced into the Fc domain.

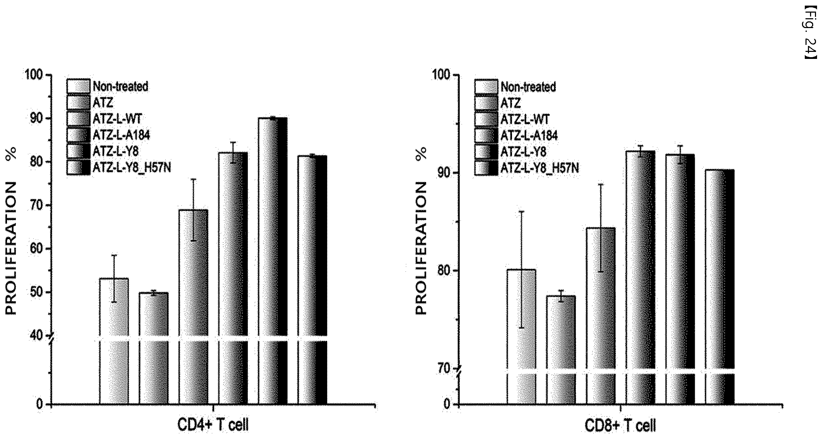

FIG. 24 is a diagram illustrating results of verifying the degree of T cell differentiation induced by proteins (50 nM) in which the light chain of Atezolizumab is fused with wild-type ICOS-L, ICOS-L A184 variant, or ICOS-L variants Y8 and Y8_H57N of the present disclosure by a flow cytometer:
   ATZ: Atezolizumab with LALAPG mutation introduced into an Fc domain;
   ATZ(L)-Wild-type: Protein in which the wild-type ICOS-L is fused to the light chain of Atezolizumab with LALAPG mutation introduced into the Fc domain;
   ATZ(L)-A184: Protein in which the ICOS-L A184 variant is fused to the light chain of Atezolizumab with LALAPG mutation introduced into the Fc domain;
   ATZ(L)-Y8: Protein in which the ICOS-L Y8 variant is fused to the light chain of Atezolizumab with LALAPG mutation introduced into the Fc domain; and
   ATZ(L)-Y8_H57N: Protein in which the ICOS-L Y8_H57N variant is fused to the light chain of Atezolizumab with LALAPG mutation introduced into the Fc domain.

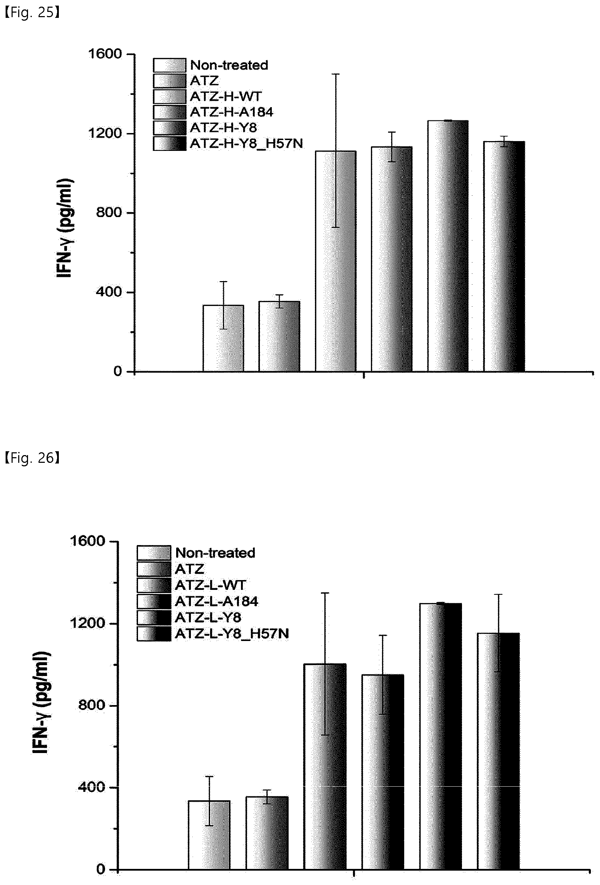

FIG. 25 is a diagram illustrating results of verifying interferon gamma secretion amounts of T cells induced by proteins (100 nM) in which the heavy chain of Atezolizumab is fused with wild-type ICOS-L, ICOS-L A184 variant, or ICOS-L variants Y8 and Y8_H57N of the present disclosure by ELISA:
   ATZ: Atezolizumab with LALAPG mutation introduced into an Fc domain;
   ATZ(H)-Wild-type: Protein in which the wild-type ICOS-L is fused to the heavy chain of Atezolizumab with LALAPG mutation introduced into the Fc domain;
   ATZ(H)-A184: Protein in which the ICOS-LA184 variant is fused to the heavy chain of Atezolizumab with LALAPG mutation introduced into the Fc domain;
   ATZ(H)-Y8: Protein in which the ICOS-L Y8 variant is fused to the heavy chain of Atezolizumab with LALAPG mutation introduced into the Fc domain; and
   ATZ(H)-Y8_H57N: Protein in which the ICOS-L Y8_H57N variant is fused to the heavy chain of Atezolizumab with LALAPG mutation introduced into the Fc domain.
FIG. 26 is a diagram illustrating results of verifying interferon gamma secretion amounts of T cells induced by proteins (100 nM) in which the light chain of Atezolizumab is fused with wild-type ICOS-L, ICOS-L A184 variant, or ICOS-L variants Y8 and Y8_H57N of the present disclosure by ELISA:
   ATZ: Atezolizumab with LALAPG mutation introduced into an Fc domain;
   ATZ(L)-Wild-type: Protein in which the wild-type ICOS-L is fused to the light chain of Atezolizumab with LALAPG mutation introduced into the Fc domain;
   ATZ(L)-A184: Protein in which the ICOS-L A184 variant is fused to the light chain of Atezolizumab with LALAPG mutation introduced into the Fc domain;
   ATZ(L)-Y8: Protein in which the ICOS-L Y8 variant is fused to the light chain of Atezolizumab with LALAPG mutation introduced into the Fc domain; and
   ATZ(L)-Y8_H57N: Protein in which the ICOS-L Y8_H57N variant is fused to the light chain of Atezolizumab with LALAPG mutation introduced into the Fc domain.

### [Best Mode of the Invention]

Hereinafter, exemplary embodiments of the present disclosure will be described in detail with reference to the accompanying drawings. However, the following exemplary embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

Terminologies used herein are terminologies used to properly express preferred exemplary embodiments of the present disclosure, which may vary according to a user, an operator's intention, or customs in the art to which the present disclosure pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present disclosure, unless otherwise defined, have the meaning as commonly understood by those skilled in the related art of the present disclosure. In addition, although preferred methods and samples are described herein, similar or equivalent methods and samples thereto are also included in the scope of the present disclosure. The contents of all publications disclosed as references in this specification are incorporated in the present disclosure.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned herein as abbreviations are also described as follows according to the IUPAC-IUB nomenclature.

Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

In one aspect, the present disclosure relates to an ICOS-L variant in which any one or more amino acids selected from the group consisting of amino acids at positions 4, 51, 52, 54, 57, 74, 130, and 234 in an amino acid sequence of wild-type ICOS-L (Inducible Co-Stimulator-ligand) are substituted with sequences different from those of the wild type.

In an exemplary embodiment, the ICOS-L variant may have enhanced binding affinity for ICOS compared to wild-type ICOS-L.

In an exemplary embodiment, the amino acids of the wild-type ICOS-L may include an amino acid sequence of SEQ ID NO: 1, and the amino acid position may be based on the amino acid sequence of SEQ ID NO: 1.

In an exemplary embodiment, the ICOS-L variant may consist of an amino acid sequence that exhibits at least 90%, 91%%, 92%, 93%, 94%, 95%, 96%, 97%, 98%, or 99% sequence identity to the amino acid sequence of SEQ ID NO: 1.

In an exemplary embodiment, the ICOS-L variant of the present disclosure may include any one or more amino acid substitutions selected from the group consisting of E4D, Q51P, Q51H, N52S, S54N, N57H, L74R, S130C and K234R, thereby enhancing binding affinity for ICOS.

In an exemplary embodiment, the ICOS-L variant of the present disclosure may include an amino acid substitution N57H and may be a variant Y8_P51Q including an amino acid sequence of SEQ ID NO: 2.

In an exemplary embodiment, the ICOS-L variant of the present disclosure may include an amino acid substitution Q51P and may be a variant Y8_H51N including an amino acid sequence of SEQ ID NO: 3.

In an exemplary embodiment, the ICOS-L variant of the present disclosure may include amino acid substitutions Q51P and N57H and may be a variant Y8 including an amino acid sequence of SEQ ID NO: 4.

In an exemplary embodiment, the ICOS-L variant of the present disclosure may include amino acid substitutions Q51H, S54N and S130C and may be a variant Y13 including an amino acid sequence of SEQ ID NO: 5.

In an exemplary embodiment, the ICOS-L variant of the present disclosure may include amino acid substitutions N52S and K234R and may be a variant Y20 including an amino acid sequence of SEQ ID NO: 6.

In an exemplary embodiment, the ICOS-L variant of the present disclosure may include amino acid substitutions E4D and Q51P and may be a variant Y35 including an amino acid sequence of SEQ ID NO: 7.

In an exemplary embodiment, the ICOS-L variant of the present disclosure may include amino acid substitutions Q51P and L74R and may be a variant Y48 including an amino acid sequence of SEQ ID NO: 8.

In an exemplary embodiment, the ICOS-L variant of the present disclosure may further include a transmembrane domain.

In an exemplary embodiment, the ICOS-L variant of the present disclosure may further include a cytoplasmic signaling domain linked to the transmembrane domain.

In an exemplary embodiment, the ICOS-L variant may be labeled with one selected from the group consisting of a chromogenic enzyme, a radioactive isotope, a chromophore, a luminescent material and a fluorescent material. The fluorescent material may be a cyanine (Cy)-based, Rhodamine-based, Alexa-based, BODIPY-based, or ROX-based fluorescent material, and may be Nile Red, BODIPY (4,4-difluoro-4-bora-3a,4a-diaza-s-indacene), cyanine, fluorescein, rhodamine, coumarin or Alexa.

The ICOS-L variant of the present disclosure refers to substitution of some amino acid sequences in the wild-type ICOS-L protein (or peptide), and as used herein, the term "variant" refers to a corresponding amino acid sequence including at least one amino acid difference (substitution, insertion, or deletion) compared to a reference substance. In specific exemplary embodiments, the "variant" has high amino acid sequence homology and/or conservative amino acid substitution, deletion and/or insertion compared to a reference sequence. In addition, as used herein, the term "ICOS-L variant" refers to an ICOS-L variant protein mutated at one or more amino acids to modulate the binding activity for ICOS thereof.

Specifically, the ICOS-L variant of the present disclosure may be prepared by a standard synthetic method, a recombinant expression system, or any other methods in the art. Accordingly, peptides according to the present disclosure may be synthesized by many methods including, for example, the following methods:
(a) a method of synthesizing a peptide by stages or by fragment assembly by means of a solid phase or liquid phase method and isolating and purifying a final peptide product;
(b) a method of expressing a nucleic acid construct encoding the peptide in a host cell, and recovering an expression product from a host cell culture; or
(c) a method of performing the expression of the nucleic acid construct encoding the peptide within a cell-free test tube, and recovering an expression product; or
a method of obtaining fragments of the peptide in any combination of (a), (b) and (c), and then linking the fragments to obtain a peptide, and recovering the corresponding peptide.

As a more specific example, through genetic engineering, a gene encoding the ICOS-L variant of the present disclosure may be prepared, transformed into a host cell, and then expressed to produce the ICOS-L variant of the present disclosure.

In one aspect, the present disclosure relates to an immunomodulatory protein including an ICOS-L variant of the present disclosure and a half-life extending moiety.

In an exemplary embodiment, the ICOS-L variant may be linked directly to the half-life extending moiety or indirectly via a linker.

In an exemplary embodiment, the half-life extending moiety may include a multimerization domain, albumin, an albumin-binding polypeptide, Pro/Ala/Ser (PAS), a C-terminal peptide (CTP) of a beta subunit of human chorionic gonadotrophin, polyethylene glycol (PEG), an unstructured long hydrophilic sequence (XTEN) of amino acid, hydroxyethyl starch (HES), an albumin-binding small molecule, or a combination thereof.

In an exemplary embodiment, the multimerization domain may be an Fc domain of immunoglobulin, a leucine zipper, an isoleucine zipper or a zinc finger.

In an exemplary embodiment, the immunomodulatory protein of the present disclosure may include the ICOS-L variant and an Fc domain of immunoglobulin or a variant thereof.

In an exemplary embodiment, the Fc domain variant may include one or more amino acid substitutions in an Fc domain of wild-type human IgG1.

In an exemplary embodiment, the Fc domain variant may exhibit a reduced effector function compared to the Fc of wild-type human IgG1, and may be an inactive or effectorless Fc.

In an exemplary embodiment, the effector function may be antibody-dependent cellular cytotoxicity (ADCC), complement-dependent cytotoxicity, programmed cell death, or cellular phagocytosis.

In an exemplary embodiment, in the Fc domain variant, amino acids at position 234, 235 or 329, numbered according to the Kabat numbering system, in the Fc domain of wild-type human IgG1, may be substituted with a sequence different from amino acids of the wild-type, and may include amino acid substitutions of L234A, L235A and P329G.

In one aspect, the present disclosure relates to a conjugate including the ICOS-L variant or the immunomodulatory protein of the present disclosure, and a targeting moiety.

In an exemplary embodiment, the ICOS-L variant or the immunomodulatory protein may be linked directly to the targeting moiety or indirectly via a linker.

In an exemplary embodiment, the targeting moiety may specifically bind to a molecule on the surface of an immune cell, and the immune cell may be an antigen presenting cell or a lymphocyte.

In an exemplary embodiment, the targeting moiety may be a tumor-localizing moiety that binds to a molecule on the tumor surface.

In an exemplary embodiment, the targeting moiety may be specific to molecules HER1/EGFR, HER2/ERBB2, CD20, CD25 (IL-2Rα receptor), CD33, CD52, CD133, CD206, CEA, CEACAM1, CEACAM3, CEACAM5, CEACAM6, cancer antigen 125 (CA125), alpha-fetoprotein (AFP), Lewis Y, Tag72, Caprin-1, Mesothelin, PDGF receptor (PDGFR; e.g., PDGF-R α), PD-1, PD-L1, CTLA-4, IL-2 receptor, vascular endothelial growth factor (VEGF), CD30, EpCAM, EphA2, glypican-3, gpA33, mucin, CAIX, PSMA, folate-binding protein, gangliosides (e.g., GD2, GD3, GM1 and GM2), VEGF receptor (VEGFR), VEGFR2, VEGF-A, integrin αVβ3, integrin α5β1, ERBB3, MET, IGF1R, EPHA3, TRAILR1, TRAILR2, RANKL, FAP, tenascin, AFP, BCR complex, CD3, CD18, CD44, CTLA-4, gp72, HLA-DR 10 β, HLA-DR antigen, IgE, MUC-1, nuC242, PEM antigen, metalloproteinase, ephrin receptor, ephrin ligand, HGF receptor, CXCR4, CXCR4, bombesin receptor, SK-1 antigen, Bcr-abl, RET, MET, TRKB, TIE2, ALK, ROS, EML4-ALK, ROS1, BRAFV600E, SRC, c-KIT, mTOR, TSC1, TSC2, BTK, KIT, BRCA, CDK 4/6, JAK1, JAK2, BRAF, FLT-3, MEK1, MEK2, SMO or B7-H6 (NCR3LG1) or bind to these components.

In an exemplary embodiment, the targeting moiety may be a protein, a peptide, a nucleic acid, a small molecule or a nanoparticle, more preferably an antibody or an immunologically active fragment thereof.

In an exemplary embodiment, the antibody may be cetuximab, panitumumab, zalutumumab, nimotuzumab, trastuzumab, ado-trastuzumab, emtacin, tositumomab (Bexxar), rituximab (Rituxan, MabThera), ibritumomab tiuxetan (Zevalin), daclizumab (Zenapax), gemtuzumab (Mylotarg), alemtuzumab, CEA-scan Fab fragment, OC125 monoclonal antibody, ab75705, B72.3, bevacizumab (Avastin), afatinib, axitinib, bosutinib, cabozatinib, ceritinib, crizotinib, dabrafenib, dasatinib, dinutuximab, erlotinib, everolimus, ibrutinib, imatinib, lapatinib, lenvatinib, nilotinib, olaparib, olaratumab, palbociclib, Pazopanib, pertuzumab, ramucirumab, regorafenib, ruxolitinib, sorafenib, sunitinib, temsirolimus, trametinib, vandetanib, vemurafenib, vismodegib, basiliximab, ipilimumab, nivolumab, pembrolizumab, MPDL3280A, pidilizumab (CT-011), AMP-224, MSB001078C, or MEDI4736, BMS-935559, LY3300054, Atezolizumab, avelumab, or durvalumab, or variants thereof, and may be Atezolizumab or variants thereof.

In an exemplary embodiment, the immunologically active fragment may be any one selected from the group consisting of Fab, Fd, Fab', dAb, F(ab'), F(ab')2, a single chain fragment variable (scFv), Fv, a single chain antibody, a Fv dimmer, a complementarity determining region fragment, a humanized antibody, a chimeric antibody, and a diabody.

In an exemplary embodiment, the ICOS-L variant of the present disclosure is linked directly or indirectly via a linker to the C-terminal or N-terminal of a heavy or light chain of an antibody or an immunologically active fragment thereof.

In an exemplary embodiment, the conjugate of the present disclosure may be a fusion protein.

In an exemplary embodiment, the conjugate of the present disclosure may further include a label for detection or purification.

In an exemplary embodiment, the conjugate of the present disclosure may also be used as a theranostic agent for the diagnosis of cancer because an antibody of the conjugate or an immunologically active fragment thereof specifically binds to a cancer cell surface molecule (e.g., PD-L1), and the ICOS-L variant of the conjugate binds to ICOS of T cells present in the tumor micro-environment to activate T cells, thereby exhibiting anticancer activity through cancer cell death by immune cells and specifically binding to cancer cells. In addition, chimeric antigen receptor (CAR)-T cells may be prepared to be used as an anticancer agent, and may also be used as an anticancer adjuvant administered simultaneously, separately or sequentially with the anticancer agent.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

The antibody or the immunologically active fragment thereof of the present disclosure may be selected from the group consisting of animal-derived antibodies, chimeric antibodies, humanized antibodies, human antibodies, and immunologically active fragments thereof. The antibody may be produced recombinantly or synthetically.

Animal-derived antibodies produced by immunizing an animal to be immunized with a desired antigen may generally cause immune rejection when administered to humans for treatment, and chimeric antibodies have been developed to suppress such immune rejection. The chimeric antibody is obtained by substituting a constant region of an animal-derived antibody, which causes an anti-isotype response, with a constant region of a human antibody using a genetic engineering method. Compared to animal-derived antibodies, the chimeric antibody is improved significantly in the anti-isotype response, but includes side effects on potential anti-idiotypic responses because animal-derived amino acids are still present in a variable region. The humanized antibody is developed to improve these side effects. The humanized antibody is produced by grafting complementarity determining regions (CDRs), which play an important role in antigen binding in the variable regions of the chimeric antibody, to a human antibody framework.

In the CDR grafting technology for fabricating the humanized antibody, it is most important to select an optimized human antibody that may best accept the CDR region of the animal-derived antibody, and to this end, applications of an antibody database, analysis of a crystal structure, molecular modeling technology, and the like are used. However, even if the CDR region of an animal-derived antibody is grafted into an optimized human antibody framework, in some cases, there are amino acids that affect antigen binding while being located in the framework of the animal-derived antibody. As a result, since there are many cases in which antigen-binding affinity is not preserved, it is required to apply additional antibody engineering techniques to restore the antigen-binding affinity.

The antibody or the immunologically active fragment thereof may be isolated from a living body (not present in a living body) or may non-naturally occur, and for example, may be synthetically or recombinantly produced.

As used herein, the "antibody" refers to a substance produced by stimulation of an antigen in the immune system, and the type thereof is not particularly limited, and may be obtained naturally or non-naturally (e.g., synthetically or recombinantly). The antibody is advantageous for mass expression and production because of being very stable in vivo as well as ex vivo and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (γ2), gamma 3 (γ3), gamma 4 (γ4), alpha 1 (α1) and alpha 2 (α2). The light chain constant region has kappa (K) and lambda (λ) types.

As used herein, the term "heavy chain" is interpreted as meaning including all of a full-length heavy chain including a variable region domain V_{H} including an amino acid sequence having a sufficient variable region sequence to impart specificity to an antigen, three constant region domains C_{H}1, C_{H}2 and C_{H}3 and hinges, and fragments thereof. In addition, the term "light chain" is interpreted as meaning including all of a full-length light chain including a variable region domain V_{L} including an amino acid sequence having a sufficient variable region sequence to impart specificity to an antigen and a constant region domain C_{L}, and fragments thereof.

As used herein, the term "variable region or variable domain" refers to a part of an antibody molecule that exhibits many variations on the sequence while performing a function that specifically binds to an antigen, and in the variable region, there are the complementarity determining regions CDR1, CDR2 and CDR3. A framework region (FR) portion exists between the CDRs to support a CDR ring. The "complementarity determining region" is a ring-shaped region involved in antigen recognition, and the specificity of the antibody for the antigen is determined as the sequence of this region changes.

As used herein, the term "single chain fragment variable (scFv)" refers to a single-chain antibody formed by expressing only a variable region of the antibody through genetic recombination, and is a single-chain form in which the VH and VL regions of the antibody are linked to each other by a short peptide chain. The term "scFv" is intended to include an scFv fragment including an antigen-binding fragment, unless otherwise specified or otherwise understood from the context. This is obvious to those skilled in the art.

As used herein, the term "complementarity determining region (CDR)" refers to an amino acid sequence of a hypervariable region of the heavy and light chains of immunoglobulin. The heavy and light chains may include three CDRs (CDRH1, CDRH2, CDRH3 and CDRL1, CDRL2, CDRL3), respectively. The CDRs may provide key contact residues for the antibody binding to an antigen or epitope.

As used herein, the term "specifically binding" or "specifically recognizing" has the same meaning as commonly known to those skilled in the art, and means that an antigen and an antibody specifically interact with each other to cause an immunological response.

As used herein, the term "antigen-binding fragment" refers to a fragment of the entire structure of immunoglobulin, and refers to a portion of a polypeptide including a portion capable of binding with the antigen. For example, the antigen-binding fragment may be scFv, (scFv)2, scFv-Fc, Fab, Fab' or F(ab')2, but is not limited thereto. The Fab of the antigen binding fragments has a structure having variable regions of the light and heavy chains, a constant region of the light chain, and a first constant region C_{H1} of the heavy chain, and has one antigen-binding site. The Fab' is different from Fab by having a hinge region containing one or more cysteine residues in a C-terminal of the heavy chain C_{H1} domain. The F(ab')2 antibody is produced when the cysteine residues in the hinge region of Fab' form disulfide bonds. Fv is a minimal antibody fragment having only a heavy chain variable region and a light chain variable region, and a recombination technique for generating the Fv fragment is widely known in the art. The two-chain Fv has the heavy chain variable region and the light chain variable region linked via a non-covalent bond, and the single-chain Fv may generally form a dimer-like structure, such as the two-chain Fv because the variable region of the heavy chain and the variable region of the single chain are covalently linked via a peptide linker or directly linked at a C-terminal. The linker may be a peptide linker consisting of 1 to 100 or 2 to 50 any amino acids, and appropriate sequences are known in the art. The antigen binding fragments may be obtained using protease (for example, the whole antibody is restriction-cleaved with papain to obtain Fab, and cleaved with pepsin to obtain a F(ab')2 fragment), or may be produced through genetic recombination technology.

As used herein, the term "hinge region" refers to a region included in the heavy chain of the antibody, and means a region which is present between the C_{H1} and C_{H2} regions and functions to provide flexibility of the antigen binding domain in the antibody. For example, the hinge may be derived from a human antibody, and specifically, derived from IgA, IgE, or IgG, such as IgG1, IgG2, IgG3, or IgG4.

In one aspect, the present disclosure relates to a nucleic acid molecule encoding the ICOS-L variant, the immunomodulatory protein, or the conjugate of the present disclosure, a vector including the same, and a host cell including the vector.

As used herein, the term "nucleic acid molecule" refers to deoxyribonucleotide or ribonucleotide that exists in a single-stranded or double-stranded form, and includes natural nucleic acid analogs unless otherwise specified (Scheit, Nucleotide Analogs, John Wiley, New York(1980); Uhlman and Peyman, Chemical Reviews , 90:543-584(1990)).

As used herein, the term "vector" refers to any nucleic acid including a competent nucleotide sequence that is inserted into a host cell to be recombined with a host cell genome and inserted to the host cell genome or replicates spontaneously as an episome. Such a vector includes a linear nucleic acid, a plasmid, a phagemid, a cosmid, an RNA vector, a viral vector, and the like.

As used herein, the term "host cell" refers to an eukaryotic or prokaryotic cell into which one or more DNAs or vectors are introduced, and should be understood to refer to not only a specific target cell but also its progeny or potential progeny. Since a certain modification may occur in subsequent generations due to mutations or environmental influences, in fact, the progeny is not identical to a parent cell, but is still included within the scope of the term as used herein.

In one aspect, the present disclosure relates to a T-cell activator including the ICOS-L variant, the immunomodulatory protein, or the conjugate of the present disclosure.

In an exemplary embodiment, the T cell may be a cytotoxic T cell or a chimeric antigen receptor (CAR)-T cell.

The ICOS is a T cell-specific costimulatory molecule that activates T cell responses to external antigens, and the facts that the ICOS is related to the prognosis of various carcinomas have been reported. Thus, the T-cell activator of the present disclosure may be utilized not only as a therapeutic agent that enhances immune stimulation, but also as a biomarker for predicting immune checkpoint inhibitor therapeutic responses.

In one aspect, the present disclosure relates to a composition for detecting ICOS including the ICOS-L variant, the immunomodulatory protein, or the conjugate of the present disclosure.

In an exemplary embodiment, the composition for detecting ICOS may be used for observing T cells present in a tumor micro-environment.

In an exemplary embodiment, T cells present in the tumor micro-environment may be observed by verifying the presence or absence of ICOS expression in cancer tissues.

In an exemplary embodiment, the composition may detect and quantify a protein expression level of ICOS.

In one aspect, the present disclosure relates to a composition for bioimaging including the ICOS-L variant, the immunomodulatory protein, or the conjugate of the present disclosure.

In one aspect, the present disclosure relates to a polynucleotide including a nucleic acid encoding an ICOS-L variant including a transmembrane domain or an ICOS-L variant further including a cytoplasmic signaling domain linked to the transmembrane domain, and at least one nucleic acid encoding at least one chain of a recombinant antigen receptor.

In an exemplary embodiment, the recombinant antigen receptor may be a chimeric antigen receptor (CAR) or an engineered T cell receptor (TCR).

In an exemplary embodiment, the nucleic acid encoding the ICOS-L variant and at least one nucleic acid encoding at least one chain of the recombinant receptor may be separated by a nucleic acid encoding a peptide causing ribosome skipping or a self-cleaving peptide, respectively.

In an exemplary embodiment, the polynucleotide may include a nucleic acid encoding an ICOS-L variant, a nucleic acid encoding a self-cleaving peptide or a peptide causing ribosome skipping, and a nucleic acid encoding a CAR.

In an exemplary embodiment, the polynucleotide may include a nucleic acid encoding an ICOS-L variant, a nucleic acid encoding a first self-cleavage peptide or a peptide causing ribosome skipping, a nucleic acid encoding one of an engineered TCR alpha chain or an engineered TCR beta chain, a nucleic acid encoding a second self-cleavage peptide or a peptide causing ribosome skipping, and a nucleic acid encoding the other of the engineered TCR alpha chain or the engineered TCR beta chain, and the self-cleaving peptide or the peptide causing ribosome skipping may be T2A, P2A, E2A or F2A.

As used herein, the term "chimeric antigen receptor (CAR)" refers to a non-naturally occurring receptor capable of imparting specificity for a specific antigen to an immune effector cell. Typically, the CAR refers to a receptor used to transplant the specificity of a monoclonal antibody into a T cell. The CAR usually consists of an ectodomain, a transmembrane domain, and an ectodomain.

The ectodomain includes an antigen recognition region, and the transmembrane domain of the CAR is in a form linked to the ectodomain, and may be derived naturally synthetically. In the case of being derived naturally, the transmembrane domain may be derived from a membrane-binding or membrane-permeating protein, and may be a portion derived from a transmembrane domain of various proteins, such as an alpha, beta or zeta chain of the T cell receptor, CD28, CD3 epsilon, CD45, CD4, CD5, CDS, CD9, CD16, CD22, CD33, CD37, CD64, CD80, CD86, CD134, CD137, CD 154 or CD8. The sequence of such a transmembrane domain may be obtained from documents known in the art that disclose the membrane-permeating region of the membrane-permeating protein, but is not limited thereto.

In addition, when the transmembrane domain is synthesized, the transmembrane domain may mainly include hydrophobic amino acid residues such as leucine and valine, and examples thereof may be present in a transmembrane domain in which a triplet of phenylalanine, tryptophan, and valine is synthesized, but are not limited thereto. Sequence information for such a transmembrane domain may be obtained from documents known in the art for the synthesized transmembrane domain, but is not limited thereto.

In the CAR of the present disclosure, the endodomain is a part of the domain of the CAR existing within a cell, and a form linked with the transmembrane domain. The endodomain of the present disclosure may include an intracellular signaling domain characterized by causing T cell activation, preferably T cell proliferation, when an antigen binds to an antigen binding site of the CAR. The intracellular signaling domain is not particularly limited in type as long as the intracellular signaling domain is a part that transmits a signal that may lead to T cell activation when an antibody binds to an antigen binding site existing outside the cell, and may be used with various types of intracellular signaling domains. For example, the intracellular signaling domain may be an immunoreceptor tyrosine-based activation motif or ITAM, and the ITAM may be derived from CD3 zeta (ξ), FcR gamma, FcR beta, CD3 gamma, CD3 delta, CD3 epsilon, CDS, CD22, CD79a, CD79b, CD66d or FcεRIγ, but is not limited thereto.

The CAR includes a single-chain fragment variable region (scFv) of an antibody specific for a tumor-associated antigen (TAA) that is coupled to the cytoplasmic domain of a T-cell signaling molecule via a hinge and a transmembrane region. Most conventional lymphocyte activating moieties include T-cell costimulatory (e.g., CD28, CD137, OX40, ICOS, and CD27) domains in a tandem with a T-cell triggering (e.g., CD3ζ) moiety. CAR-mediated adoptive immunotherapy allows a CAR-grafted cell to directly recognize a TAA on a target tumor cell in a non-HLA-restricted manner.

In one aspect, the present disclosure relates to a vector including the polynucleotide.

In an exemplary embodiment, the vector may be a viral vector, and the viral vector may be a retroviral vector or a lentiviral vector.

In one aspect, the present disclosure relates to an engineering cell including the polynucleotide or the vector.

In one aspect, the present disclosure relates to an engineering cell including the ICOS-L variant, the immunomodulatory protein, or the conjugate of the present disclosure.

In an exemplary embodiment, the engineering cell may further include a chimeric antigen receptor (CAR) or an engineered T-cell receptor.

In an exemplary embodiment, the ICOS-L variant, the immunomodulatory protein, or the conjugate of the present disclosure may be secreted from an engineered cell, and the engineered cell may include an ICOS-L variant including a transmembrane domain.

In an exemplary embodiment, the ICOS-L variant of the present disclosure may be expressed on the cell surface.

In an exemplary embodiment, the cell may be an immune cell, and the immune cell may be an antigen presenting cell (APC) or a lymphocyte, and the lymphocyte may be a T cell. The T cell includes CD4+ T cells (helper T cells, TH cells), CD8+ T cells (cytotoxic T cells, CTLs), memory T cells, regulatory T cells (Treg cells), natural killer T cells, etc., and the T cells into which the CAR is introduced in the present disclosure are preferably CD8+ T cells, but are not limited thereto.

In an exemplary embodiment, the engineered cell may be a primary cell, and in some cases, the cells may be mammalian cells or human cells.

In an exemplary embodiment, the engineered cell may be a chimeric antigen receptor expressing cell, a chimeric antigen receptor expressing macrophage (CAR-macrophage), a chimeric antigen receptor expressing T (CAR-T) cell, a chimeric antigen receptor expressing-gamma-delta T (CAR-Gamma-delta T) cell, or a natural killer (CAR-NK) cell.

As used herein, the term "chimeric antigen receptor expressing T (CAR-T) cell" means a T cell expressing a CAR. The CAR-T cell i) recognizes a cancer antigen in a manner independent of human leukocyte antigen (HLA) to have the advantage of being able to treat cancers that evade the action of anticancer drugs by reducing HLA expression on the cell surface, ii) is independent of HLA type to be used for treatment regardless of the patient's HLA type, and iii) has the advantage of being able to produce a large number of cancer-specific T cells in a short period of time to exhibit excellent anticancer effects.

In one aspect, the present disclosure relates to an infectious agent including a nucleic acid molecule encoding the ICOS-L variant, the immunomodulatory protein, or the conjugate of the present disclosure.

In an exemplary embodiment, the ICOS-L variant, the immunomodulatory protein, or the conjugate is secreted from the infectious agent when expressed, and in some cases, the encoded ICOS-L variant may include a transmembrane domain.

In an exemplary embodiment, the encoded ICOS-L variant may be expressed on the cell surface to be expressed.

In an exemplary embodiment, the infectious agent may be bacteria or virus, and the virus may be an oncolytic virus. The oncolytic virus may be adenovirus, adeno-associated virus, herpes virus, varicella-zoster virus, vesicular stomatitis virus, reovirus, Newcastle disease virus, parvovirus, measles virus, vesicular stomatitis virus (VSV), Coxsackie virus or vaccinia virus.

In an exemplary embodiment, the virus may specifically target dendritic cells (DCs) and/or may be dendritic cell-tropic, and the virus may be a lentiviral vector pseudotyped with a modified Sindbis virus envelope product.

In an exemplary embodiment, the infectious agent may further include a nucleic acid molecule encoding another gene product capable of causing death of a target cell or initiating or boosting an immune response. The additional gene product may be selected from anticancer agents, antimetabolites, antiangiogenic agents, immunomodulatory molecules, immune checkpoint inhibitors, antibodies, cytokines, growth factors, antigens, cytotoxic gene products, pro-apoptotic gene products, antiapoptotic gene products, cell matrix degradation genes, genes for tissue regeneration or genes that reprogram human somatic cells to pluripotency.

In one aspect, the present disclosure relates to a pharmaceutical composition for preventing or treating inflammatory or autoimmune diseases, including an ICOS-L variant or immunomodulatory protein of the present disclosure as an active ingredient.

In an exemplary embodiment, the inflammatory disease may be antineutrophil cytoplasmic antibody (ANCA)-associated vasculitis, vasculitis, autoimmune skin disease, graft, rheumatic disease, inflammatory gastrointestinal disease, inflammatory ocular disease, inflammatory neurological disease, inflammatory lung disease, inflammatory endocrine disease, or autoimmune hematological disease.

In an exemplary embodiment, the autoimmune disease may be inflammatory bowel disease, Crohn's disease, ulcerative colitis, multiple sclerosis, asthma, rheumatoid arthritis or psoriasis.

In one aspect, the present disclosure relates to an anticancer adjuvant including the ICOS-L variant, the immunomodulatory protein, the conjugate or the engineered cell of the present disclosure.

In an exemplary embodiment, the adjuvant may enhance the anticancer effect of immune checkpoint inhibitors and may be administered simultaneously, separately, or sequentially with immune checkpoint inhibitors.

In an exemplary embodiment, the immune checkpoint inhibitor may be an anti-PD-1 antibody, an anti-PD-L1 antibody, an anti-CTLA-4 antibody, or a variant thereof.

In one aspect, the present disclosure relates to a pharmaceutical composition for preventing or treating cancer, comprising the ICOS-L variant, the immunomodulatory protein, the conjugate or the engineered cell of the present disclosure.

In one exemplary embodiment, the cancer may be any one or more selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

The pharmaceutical composition of the present disclosure may be used as a single therapy, but may also be used in combination with other conventional biological therapy, chemotherapy or radiation therapy, and may treat more effectively cancer in the case of such concurrent therapy. In the case of using the present disclosure for preventing or treating cancer, a chemotherapeutic agent that may be used with the composition includes cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, methotrexate, and the like. Radiation therapy that may be used together with the composition of the present disclosure includes X-ray irradiation and γ-ray irradiation.

As used herein, the term "prevention" means all actions that inhibit or delay the occurrence, spread, and recurrence of cancer by administration of the ICOS-L variant or the composition including the ICOS-L variant according to the present disclosure.

The therapeutically effective amount of the composition of the present disclosure may vary depending on many factors, for example, an administration method, a target site, a condition of a patient, and the like. Accordingly, when used in the human body, the dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. As used herein, the term 'pharmaceutically effective amount' refers to a an amount enough to treat the disease at a reasonable benefit/risk ratio applicable to medical treatment and not to cause side effects. An effective dose level may be determined according to factors including the health condition of a patient, the type and severity of a disease, the activity of a drug, the sensitivity to a drug, a method of administration, a time of administration, a route of administration, an emission rate, duration of treatment, and mixed or simultaneously used drugs, and other factors well-known in the medical field. The composition of the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include a carrier, diluent, excipient, or a combination of two or more thereof, which are commonly used in biological agents. As used herein, the term "pharmaceutically acceptable" means exhibiting non-toxic properties to normal cells or humans exposed to the composition. The carrier is not particularly limited as long as the carrier is suitable for in vivo delivery of the composition, and may be used by mixing, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In an exemplary embodiment, the pharmaceutical composition may be one or more formulations selected from the group including oral formulations, external formulations, suppositories, sterile injection solutions and sprays.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by mixing, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the composition may be prepared in injectable formulations such as an aqueous solution, a suspension, and an emulsion, pills, capsules, granules, or tablets by further adding a diluent, a dispersant, a surfactant, a binder, and a lubricant. Furthermore, the composition may be prepared preferably according to each disease or ingredient using a suitable method in the art or a method disclosed in Remington's Pharmaceutical Science (Mack Publishing Company, Easton PA, 18th, 1990).

The composition of the present disclosure may further include one or more active ingredients exhibiting the same or similar functions. The composition of the present disclosure includes 0.0001 to 10 wt%, preferably 0.001 to 1 wt% of the protein, based on the total weight of the composition.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present disclosure is preferably included in an amount of 0.1 part by weight to 90 parts by weight based on the composition, but is not limited thereto.

The composition of the present disclosure may be administered parenterally (e.g., applied intravenously, subcutaneously, intraperitoneally or topically) or orally according to a desired method, and the dose may vary depending on the weight, age, sex, and health condition of a patient, a diet, an administration time, an administration method, an excretion rate, the severity of a disease, etc. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In an exemplary embodiment, the composition of the present disclosure may be used as a combined therapeutic agent with an immune checkpoint inhibitor.

In one aspect, the present disclosure relates to a composition for diagnosing cancer, including the ICOS-L variant, the immunomodulatory protein, the conjugate or the engineered cell of the present disclosure.

In an exemplary embodiment, the composition may further include one selected from the group consisting of a chromogenic enzyme, a radioisotope, a chromophore, a luminescent material, and a fluorescent material.

In one aspect, the present disclosure relates to a kit for diagnosing cancer including a composition for diagnosing cancer of the present invention.

In an exemplary embodiment, the kit may further include not only tools and/or reagents for collecting a biological sample from a subject or patient, but also tools and/or reagents for preparing genomic DNA, cDNA, RNA or protein from the sample.

As used herein, the term 'kit for diagnosing cancer' refers to a kit including the composition for diagnosing cancer of the present disclosure. Accordingly, the expression 'kit for diagnosing cancer' can be used interchangeably or in combination with the 'composition for diagnosing cancer'. As used herein, the term 'diagnosis' includes determining the susceptibility of a subject to a specific disease or disorder, determining whether a subject currently has a specific disease or disorder, determining the prognosis of a subject suffering from a specific disease or disorder (e.g., identifying a pre-metastatic or metastatic cancer state, determining a stage of cancer, or determining the responsiveness of cancer to treatment), or therametrics (e.g., monitoring the condition of a subject to provide information about treatment efficacy).

The ICOS-L variant of the present disclosure has an anticancer activity that kills cancer cells by acting as an immunostimulatory drug that promotes the activation of T cells by specifically binding to ICOS with significantly enhanced binding affinity, or an anticancer activity that induces anticancer efficacy by killing regulatory T cells expressing ICOS, and can be used as a theranostic agent for the diagnosis of cancer by specifically binding to T cells present in the tumor micro-environment.

In one aspect, the present disclosure relates to a method for providing information for cancer diagnosis, including contacting a biological sample isolated from a subject with the composition for diagnosing cancer of the present disclosure; determining a binding level of the ICOS-L variant and ICOS; and comparing a binding level of the ICOS-L variant and ICOS in a normal control sample.

In one exemplary embodiment, when the binding level of the ICOS-L variant and PD-L1 in the biological sample isolated from the subject is higher than the binding level of the ICOS-L variant and PD-L1 in the normal control sample, the method may further include determining that the test subject is cancer.

As used herein, the term "sample" refers to a biological sample obtained from a subject or patient. Sources of the biological sample may be fresh, frozen and/or preserved organ or tissue samples or solid tissue from biopsies or aspirates; blood or any blood component; and cells at any time point of conception or development in a subject.

In one aspect, the present disclosure relates to a method for predicting therapeutic response or diagnosing prognosis for an immune checkpoint inhibitor, including confirming the expression level of ICOS using the ICOS-L variant, the immunomodulatory protein, the conjugate or the engineered cell of the present disclosure in a biological sample isolated from a subject treated with an immune checkpoint inhibitor.

In one aspect, the present disclosure relates to a use of the ICOS-L variant, the immunomodulatory protein, the conjugate or the engineered cell of the present disclosure for preventing or treating cancer.

In one aspect, the present disclosure relates to a method for treating cancer including administering the ICOS-L variant, the immunomodulatory protein, the conjugate or the engineered cell of the present disclosure in a pharmaceutically effective amount to a subject suffering with cancer.
Hereinafter, the present disclosure will be described in more detail with reference to the following Examples. However, the following Examples are only intended to embody the contents of the present disclosure, and the present disclosure is not limited thereto.

### [Modes of the Invention]

### Example 1. Preparation of dimeric human ICOS (ICOS-Fc) for screening ICOS-L variants

### 1-1. Dimeric human ICOS cloning

To screen ICOS-L variants with stronger binding affinity for ICOS more efficiently by increasing avidity, an Fc region (domain) of human IgG antibody was expressed at the C-terminal portion of ICOS to induce dimerization, and a GS linker consisting of glycine and serine was inserted between Fc and ICOS to secure the fluidity of each protein. Specifically, a gene was amplified using primers designed by Twist Bioscience (USA) by synthesizing DNA of an amino acid sequence E21-K140, which was a part of an extracellular domain of ICOS, and Vent Polymerase (New England Biolab). An Fc gene was also amplified using the designed primers and Vent Polymerase, and the amplified ICOS and Fc genes were subjected to assembly PCR, and treated with restriction enzymes using BssHII and XbaI (New England Biolab). The ICOS-Fc gene treated with the restriction enzymes was ligated into a pMAZ vector, which was a vector for animal cells treated with the same restriction enzymes. The ligated plasmid was transformed into Jude1 E. coli to obtain a monoclone and it was confirmed that the ICOS-Fc was successfully inserted into the pMAZ vector through base sequencing.

### 1-2. Expression and purification of dimeric human ICOS (ICOS-Fc)

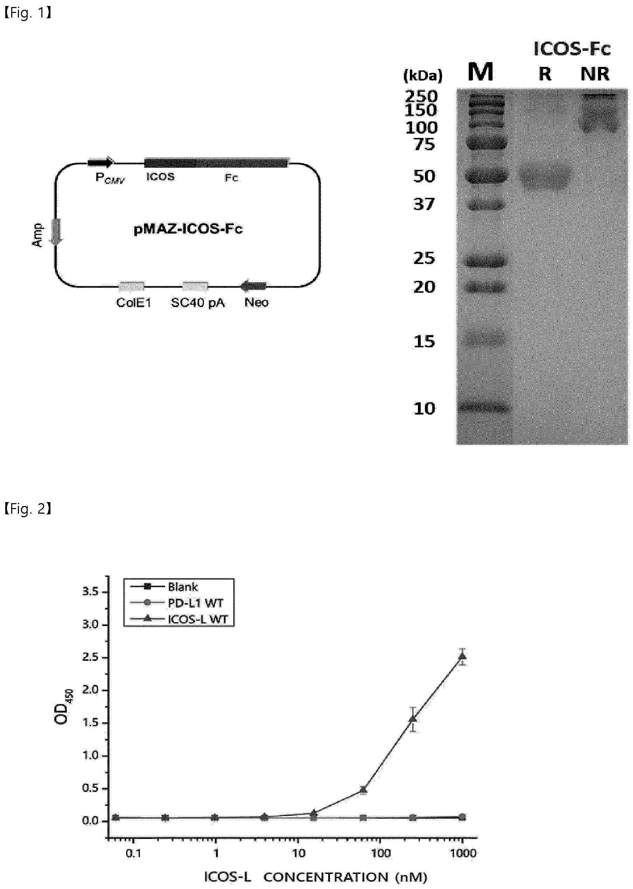

300 ml of expi293F cells were subcultured at a density of 2 × 10⁶ cells/ml, and one day later, the ICOS-Fc expression vector prepared in Example 1 was mixed with PEI (Polyehylenimine, Polyscience, 23966) in a ratio of 1:4 in 30 ml of a Freestyle 293 expression culture medium (Gibco. 12338-018), left at room temperature for 20 minutes, and then transfected into the Expi293F animal cells. The cells were incubated for 7 days under conditions of 37°C, 125 rpm, and 8% CO₂ in a CO₂ shaking incubator, and then centrifuged to collect only a supernatant. Thereafter, the supernatant was equilibrated with 25x PBS and filtered through a 0.2 µm filter (Merck Millipore) using a bottle top filter. The filtered culture solution was added with 1 ml of a Protein A resin and stirred at 4°C for 16 hours, passed through a column to recover a resin, and then washed with 10 ml of PBS. The washed resin was eluted with 100 mM of a glycine pH 2.7 buffer and then neutralized using 1 M Tris-HCl pH 8.0 to obtain a purified ICOS-Fc dimer protein, and the buffer was replaced with 1x PBS using Centrifugal filter units 3K (Merck Millipore). The expressed and purified ICOS-Fc dimer protein was confirmed by a SDS-PAGE gel (FIG. 1).

### 1-3. Validation of binding affinity of purified ICOS-Fc for ICOS-L and fluorescent labeling

To analyze the binding affinity of ICOS-Fc purified in Example 1-2 above for ICOS-L, ELISA assay was performed. Specifically, 50 µl of ICOS-Fc diluted to 4 µg/ml in 0.05 M Na₂CO₃ (pH 9.6) was dispensed into a Flat Bottom Polystyrene High Bind 96-well microplate (Costar), immobilized at 4°C for 16 hours, and then blocked with 100 µl of 0.5% bovine serum albumin (BSA) (Gibco) (in PBS, pH 7.4) for 2 hours at room temperature. After washing four times with 180 µl of 0.05% PBST (pH 7.4), 50 µl of wild-type ICOS-L serially diluted with 0.5% BSA (in PBS pH 7.4) was dispensed into each well and reacted at room temperature for 1 hour. After washing four times, 50 µl of an anti-His-HRP conjugate was added and reacted at room temperature for 1 hour, and then washed four times. Thereafter, 50 µl of a 1-Step Ultra TMB-ELISA substrate solution (Thermo Fisher Scientific) was added to develop color, and then added with 50 µl of 2 M H₂SO₄ to terminate the reaction, and analyzed using an Epoch microplate spectrophotometer (BioTek).

As a result, it was confirmed that ICOS-Fc bound to wild-type ICOS-L (FIG. 2), and the ICOS dimer protein was fluorescently labeled using an Alexa488 labeling kit for use as a probe.

### Example 2. Selection of screening method

### 2-1. ICOS-L cloning for selection of yeast surface display method

For efficient variant screening, a yeast surface anchoring motif intended to be determined and ICOS-L was cloned to compare a system for anchoring an N-terminal of ICOS-L to Aga2 (pCTCON-Aga2-ICOS-L-FLAG) and a system for anchoring a C-terminal to Aga2 (pCTCON-ICOS-L-Aga2-FLAG). Specifically, the gene was amplified using primers designed by Twist Bioscience (USA) by synthesizing a wild-type ICOS-L gene and a conventional variant A184 (N52H, N57Y, and Q100P) and Vent Polymerase (New England Biolab). The amplified wild-type ICOS-L and A184 genes were treated with a restriction enzyme using SfiI (New England Biolab), and the restriction enzyme-treated genes were ligated into a pCTCON vector treated with the same restriction enzyme. The ligated plasmid was transformed into Jude1 E. coli, and monoclones were obtained and sequenced to confirm that four plasmids, pCTCON-Aga2-ICOS-L_WT-FLAG, pCTCON-Aga2-ICOS-L_A184-FLAG, pCTCON-ICOS-L_WT-Aga2-FLAG, and pCTCON-ICOS-L_ A184-Aga2-FLAG, had been successfully cloned.

### 2-2. Selection of display method

A yeast display method was selected through verification of binding affinity for ICOS, and each of the four plasmids prepared in Example 2-1 was transformed into a AWY101 (Trp-) strain. To confirm the binding affinity of ICOS-L for ICOS using a flow cytometer, the strain was cultured in 5 ml of a SDCAA medium (20 g/L Glucose, 6.7 g/L Yeast nitrogen base without amino acids, 5 g/L casamino acids, 5.4 g/L Na₂HPO₄, 8.56 g/L NaH₂PO₄) supplemented with 50 µg/ml Kanamycin and 40 µg/ml Chloramphenicol at 30°C and 225 rpm for 16 hours. The cultured cells were centrifuged (2,500 g, 5 minutes, 4°C) to obtain 5 × 10⁷ cells, and then induced in 5 ml of a SGCAA medium (20 g/L Glucose, 6.7 g/L Yeast nitrogen base without amino acids, 5 g/L casamino acids, 5.4 g/L Na₂HPO₄, 8.56 g/L NaH₂PO₄) supplemented with 50 µg/ml Kanamycin and 40 µg/ml Chloramphenicol at 20°C and 225 rpm for 24 hours. After induction, 2 × 10⁷ cells were collected in an e-tube by centrifugation (14,000 g, 30 s, 4°C). Each e-tube with the collected cells was added with 1 ml of PBSB (0.1% BSA in PBS), resuspended, centrifuged (14,000 g, 30 sec, 4°C) to collect the cells again, and then resuspended in 0.5 ml of PBSB to prepare 4 × 10⁷ cells/ml. 25 µl of cells were transferred to a new e-tube, and then added with 25 µl of PBSB, ICOS-Fc-Alexa488 (200 nM) and an Anti-FLAG-iFluor647 (1000:1) probe, respectively, and incubated at room temperature for 30 minutes to label the cells with fluorescent probes. Thereafter, the cells were centrifuged (14,000 g, 30 sec, 4°C) to discard a supernatant and resuspended and washed in 200 µl of PBSB, and then centrifuged again (14,000 g, 1 min, 4°C) and resuspended in 200 µl of PBSB to prepare samples. The expression levels of wild-type ICOS-L and A184 and binding affinity for ICOS were indirectly analyzed by measuring the fluorescence signal values of the samples prepared with a FACSLyric (BD Biosciences) device.

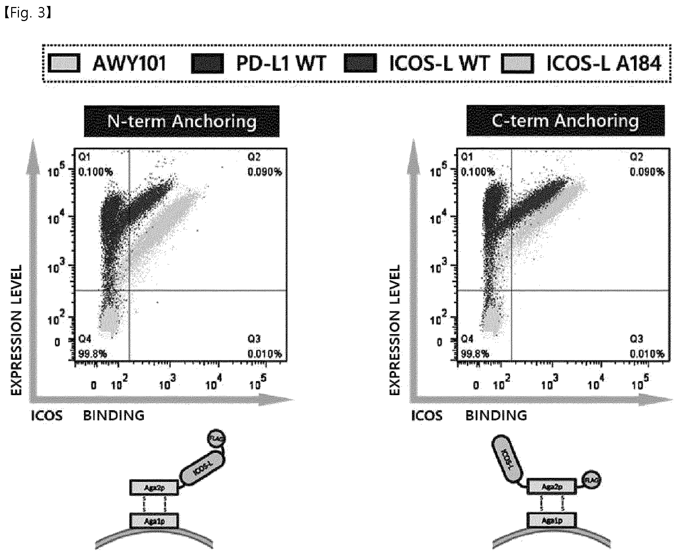

As a result, all of above plasmids were expressed well, but it was confirmed that the binding affinity of ICOS-L displayed in yeast for ICOS was significantly increased by anchoring the C-terminal portion to Aga2 compared to that of wild-type ICOS-L displayed in yeast by anchoring the N-terminal portion to Aga2, and thus it was determined to proceed with screening in the form of anchoring the C-terminal (FIG. 3).

### Example 3. Construction of large ICOS-L error-prone library for using ultra-fast screening technique

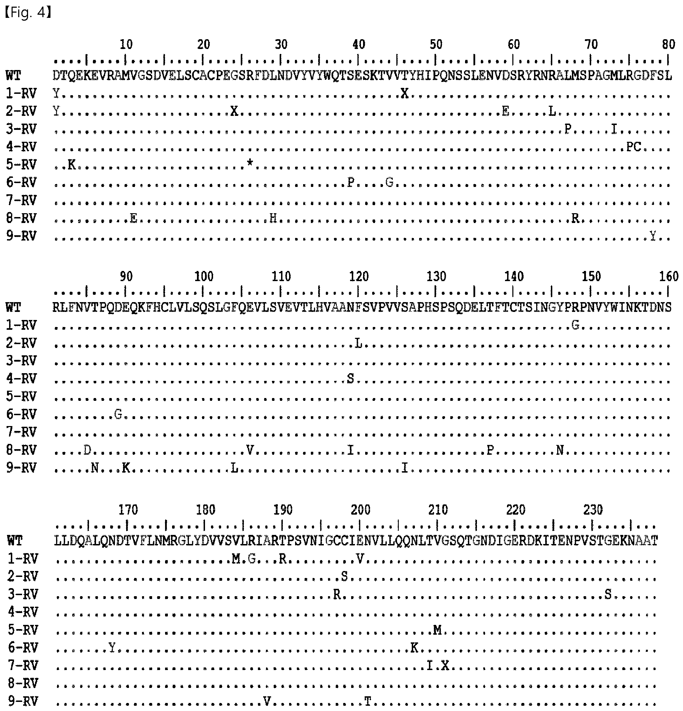

To rapidly explore ICOS-L variants with increased binding affinity for ICOS, based on pCTCON-ICOS-L_WT-Aga2-FLAG, primers containing both SfiI sites were designed to allow random mutations in all regions of ICOS-L. DNA was first amplified by Error Prone PCR technique using the designed primers, Taq Polymerase (TAKARA), dNTPs (Invitrogen), MgCl₂, and MnCl₂ (SIGMA). The amplified gene was prepared by second amplification using Vent polymerase (24 µg), and the corresponding vector was prepared by treatment with a SfiI restriction enzyme (8 µg). The two prepared genes were transformed into a AWY101 strain to construct a library through homologous recombination. The constructed library had a size of 4.5 × 10⁷ and was confirmed through sequencing to have an error rate of 0.89% (average of 6.3 mutations/total 714 bp) based on DNA and 2.05% (average of 4.8 mutations/total 238 amino acids) based on amino acids (FIG. 4).

### Example 4. ICOS-L variant screening

### 4-1. ICOS-L variant screening

An initial library transformed into AWY101 was cultured in 500 ml of a SDCAA medium (20 g/L Glucose, 6.7 g/L Yeast nitrogen base without amino acids, 5 g/L casamino acids, 5.4 g/L Na₂HPO₄, 8.56 g/L NaH₂PO₄) supplemented with 50 µg/ml Kanamycin and 40 µg/ml Chloramphenicol at 30°C and 225 rpm for 16 hours. To filter out dead cells, the cultured cells were inoculated into 100 ml of the SDCAA medium at OD₄₅₀ = 0.7 and then cultured at 30°C and 225 rpm for 16 hours, and the cultured cells were inoculated into 100 ml of the SGCAA medium at OD₄₅₀ = 0.7 and cultured at 20°C and 225 rpm for 2 days. After induction, 1 × 10⁸ cells were collected in an e-tube by centrifugation (14,000 g, 30 sec, 4°C). The e-tube with the collected cells was added with 1 ml of PBSB (0.1% BSA in PBS), resuspended, centrifuged (14,000 g, 30 sec, 4°C) to collect the cells again, and then resuspended with 1 ml of PBSB added with Anti-FLAG-iFluor647 (1000:1) and ICOS-Fc-Alexa488 (200 nM) probes, and incubated at room temperature for 1 hour to be labeled. Thereafter, the cells were centrifuged (14,000 g, 30 sec, 4°C) to discard a supernatant and resuspended and washed in 1 ml of PBSB, and then centrifuged again (14,000 g, 1 min, 4°C) and responded in 1 ml of PBSB to prepare samples. The fluorescence signal values of the prepared library samples were measured using S3 sorter (Bio-Rad) equipment to recover yeasts with high binding affinity for ICOS, and the recovered yeasts were cultured in 20 ml of SDCAA at 30°C and 225 rpm. The cultured cells were recovered the next day and cultured in 100 ml of SGCAA at 20°C and 225 rpm for 2 to 3 days, and after induction, the next round was performed. The screening process was performed a total of 4 times while reducing the concentration of the probe.

### 4-2. Confirmation of amplification of ICOS-L variants with enhanced binding affinity for ICOS

Initial, Round 1, Round 2, Round 3 and Round 4 libraries were separately inoculated in 100 ml of a SDCAA medium (20 g/L Glucose, 6.7 g/L Yeast nitrogen base without amino acids, 5 g/L casamino acids, 5.4 g/L Na₂HPO₄, 8.56 g/L NaH₂PO₄) supplemented with 50 µg/ml Kanamycin and 40 µg/ml Chloramphenicol and cultured at 30°C and 225 rpm for 16 hours. To filter out dead cells, the cultured cells were inoculated into 100 ml of the SDCAA medium at OD₄₅₀ = 0.7 and then cultured at 30°C and 225 rpm for 16 hours, and the cultured cells were inoculated into 100 ml of the SGCAA medium at OD₄₅₀ = 0.7 and cultured at 20°C and 225 rpm for 2 days. After induction, the libraries were centrifuged (14,000 g, 30 sec, 4°C) in an amount corresponding to 2 × 10⁷ cells and collected in an e-tube. Each e-tube with the collected cells was added with 1 ml of PBSB (0.1% BSA in PBS), resuspended, centrifuged (14,000 g, 30 sec, 4°C) to collect the cells again, and then resuspended in 0.5 ml of PBSB to prepare 4 × 10⁷ cells/ml. 25 µl of cells were transferred to a new e-tube, and then added with 25 µl of PBSB, ICOS-Fc-Alexa488 (200 nM) and an Anti-FLAG-iFluor647 (1000:1) probe, respectively, and incubated at room temperature for 30 minutes to label the cells with fluorescent probes. Thereafter, the cells were centrifuged (14,000 g, 30 sec, 4°C) to discard a supernatant, resuspended and washed in 200 µl of PBSB, and then centrifuged again (14,000 g, 1 min, 4°C) and resuspended in 200 µl of PBSB to prepare samples. The binding affinity of each library for ICOS was indirectly analyzed by measuring the fluorescence signal values of the prepared samples with a FACSLyric (BD Biosciences) device.

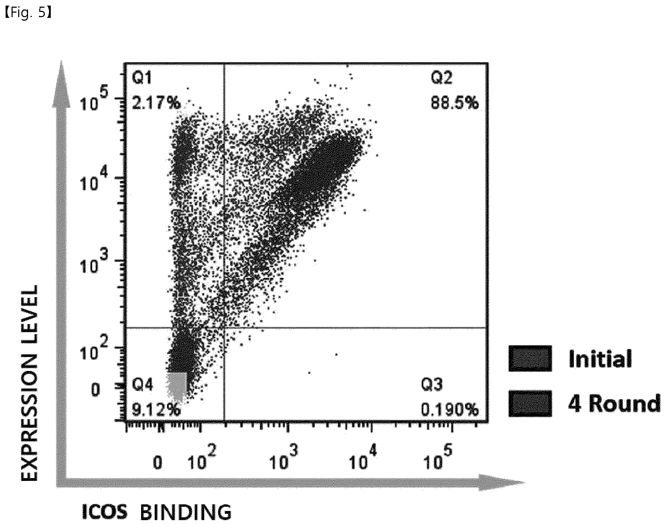

As a result, it was confirmed that as the screening progressed, variants with enhanced binding affinity for ICOS were gradually enriched (FIG. 5).

### Example 5. Amino acid sequencing of ICOS-L variants and screening of ICOS-L variants with enhanced binding affinity for ICOS

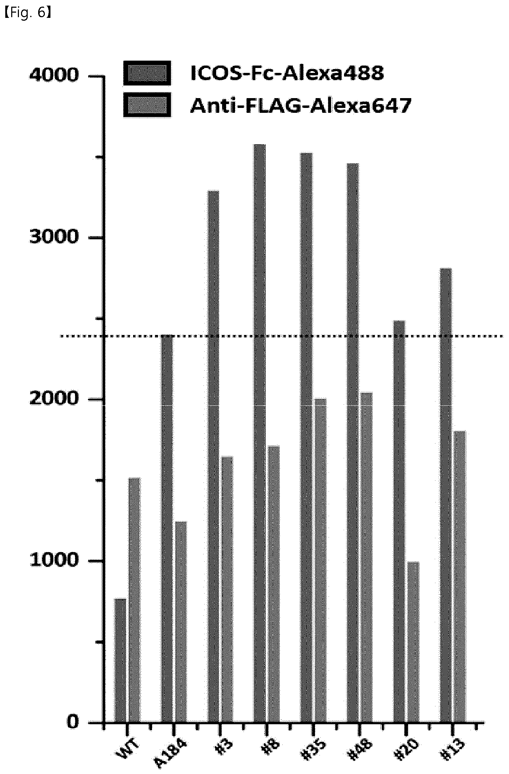

DNA from the 4 Round library was obtained using Zymoprep Yeast Plasmid Miniprep kits (Zymo Research), and then transformed into Jude1 E. coli, and sequencing for 50 colonies was performed. As a result, 18 variants were selected, and the plasmids of the 18 variants were each transformed into a AWY101 (Trp-) strain to analyze their binding affinity for ICOS. To confirm the binding affinity of the variants for ICOS using a flow cytometer, wild-type ICOS-L, A184 and 18 variants were cultured in 5 ml of a SDCAA medium (20 g/L Glucose, 6.7 g/L Yeast nitrogen base without amino acids, 5 g/L casamino acids, 5.4 g/L Na₂HPO₄, 8.56 g/L NaH₂PO₄) supplemented with 50 µg/ml Kanamycin and 40 µg/ml Chloramphenicol at 30°C and 225 rpm for 16 hours. The cultured cells were centrifuged (2,500 g, 5 min, 4°C) to obtain 5 × 10⁷ cells, and then induced in 5 ml of a SGCAA medium (20 g/L Glucose, 6.7 g/L Yeast nitrogen base without amino acids, 5 g/L casamino acids, 5.4 g/L Na₂HPO₄, 8.56 g/L NaH₂PO₄) supplemented with 50 µg/ml Kanamycin and 40 µg/ml Chloramphenicol at 20°C and 225 rpm for 24 hours. After induction, 2 × 10⁷ cells were collected in an e-tube by centrifugation (14,000 g, 30 sec, 4°C). Each e-tube with the collected cells was added with 1 ml of PBSB (0.1% BSA in PBS), resuspended, centrifuged (14,000 g, 30 sec, 4°C) to collect the cells again, and then resuspended in 0.5 ml of PBSB to prepare 4 × 10⁷ cells/ml. 25 µl of cells were transferred to a new e-tube, and then added with 25 µl of PBSB, ICOS-Fc-Alexa488 (67.7 nM) and an Anti-FLAG-iFluor647 (1000:1) probe, respectively, and incubated at room temperature for 30 minutes to label the cells with fluorescent probes. Thereafter, the cells were centrifuged (14,000 g, 30 sec, 4°C) to discard a supernatant, resuspended and washed in 200 µl of PBSB, and then centrifuged again (14,000 g, 1 min, 4°C) and resuspended in 200 µl of PBSB to prepare samples. The expression levels of the variants and the binding affinity for ICOS were indirectly analyzed by measuring the fluorescence signal values of the prepared samples with a FACSLyric (BD Biosciences) device. Through this, a total of six ICOS-L variants Y3, Y8, Y13, Y20, Y35, and Y48 with enhanced binding affinity for ICOS were selected (FIG. 6).

### Example 6. Expression and purification of ICOS-L variants with enhanced binding affinity for ICOS

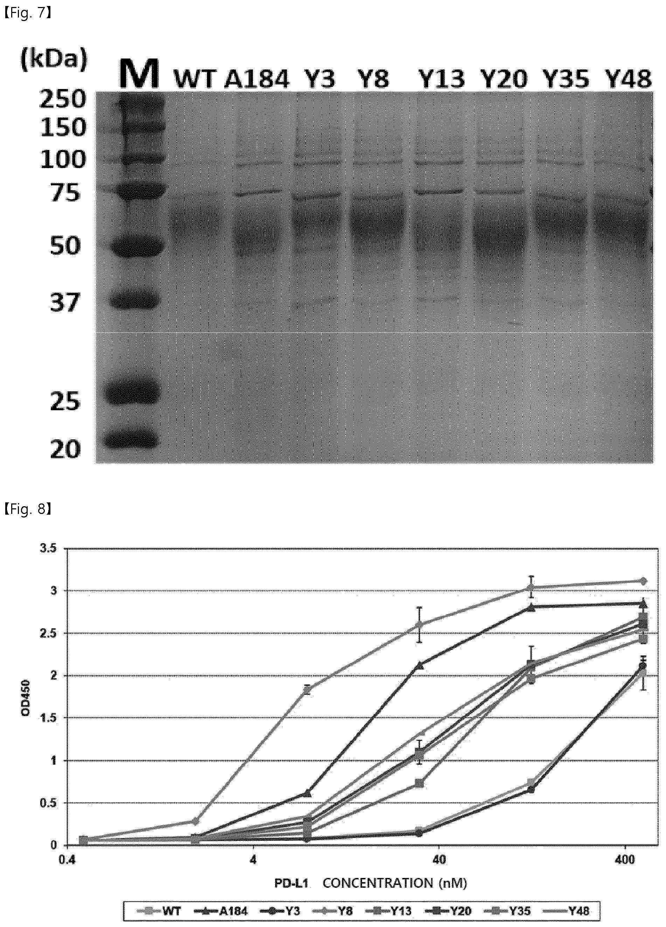

To construct animal cell expression vectors for ICOS-L variants, wild-type ICOS-L, a control ICOS-L_A184, and Y3, Y13, Y20, Y35, and Y48 of six variant genes obtained in Example 5 were amplified using designed primers and Vent Polymerase (New England Biolab). The amplified genes were treated with restriction enzymes using BssHII and XbaI (New England Biolab). The genes of the ICOS-L variants treated with the restriction enzymes were ligated into a pMAZ vector, which was a vector for animal cells treated with the same restriction enzymes. The ligated plasmids were transformed into Jude1 E. coli, and then monoclones were obtained and sequencing was performed, and as a result, it was confirmed that the genes of the variants were successfully inserted into the vector. In addition, the Y8 variant had an XbaI restriction enzyme site within the gene sequence and thus was amplified using the designed primers and Vent Polymerase, and then inserted into the pMAZ vector using a 2X Gibson assembly master mix (New England Biolab). After the reaction was completed, the gene sample was transformed into Jude1 E. coli, and then monoclones were obtained and sequencing was performed, and as a result, it was confirmed that the Y8 gene was also successfully inserted into the vector. The Expi293F animal cells were transfected with the constructed ICOS-L variant expression vectors (pMAZ-ICOS-L_WT, pMAZ-ICOS-L_A184, pMAZ-ICOS-L_Y3, pMAZ-ICOS-L_Y8, pMAZ-ICOS-L_Y13, pMAZ-ICOS-L_Y20, pMAZ-ICOS-L_Y35, and pMAZ-ICOS-L_Y48), cultured for 7 days in a CO₂ shaking incubator under conditions of 37°C, 125 rpm, and 8% CO₂, and then centrifuged to separate only the supernatant. Thereafter, the culture medium was equilibrated using 25x PBS and filtered using a 0.2 µm syringe filter (Sartorius, S6634). The filtered culture medium was added with 0.15 ml of Ni-NTA resin, stirred at room temperature for 1 hour to recover the resin, and washed twice with 1 ml of a PBS solution containing 10 mM imidazole (Sigma) and washed twice more with 1 ml of PBS solution containing 20 mM imidazole. The culture medium was eluted with a PBS solution containing 250 mM imidazole, and then the buffer was changed to 1X PBS (pH 7.4) using centrifugal filter units 10K (Merck Millipore). Thereafter, the expressed and purified wild-type ICOS-L, control A184, and six variants discovered in the present disclosure were confirmed using SDS-PAGE gel (FIG. 7).

### Example 7. Verification of binding affinity of ICOS-L variants for ICOS

ELISA was performed to analyze the ICOS binding affinity of purified ICOS-L variants. Specifically, 50 µl of ICOS-Fc diluted to 4 µg/ml in 0.05 M Na₂CO₃ (pH 9.6) was dispensed into a Flat Bottom Polystyrene High Bind 96-well microplate (Costar), immobilized at 4°C for 16 hours, and then blocked with 100 µl of 0.5% BSA (Gibco) (in PBS, pH 7.4) for 2 hours at room temperature. After washing four times with 180 µl of 0.05% PBST (pH 7.4), 50 µl of ICOS-L variants serially diluted with 0.5% BSA (in PBS pH 7.4) were dispensed into each well and reacted at room temperature for 1 hour. After washing four times, 50 µl of an anti-His-HRP conjugate was added and reacted at room temperature for 1 hour, and then washed four times. Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction, and then analyzed using an Epoch microplate spectrophotometer (BioTek).

As a result, all of the variants except for the Y3 variant showed higher binding affinity for ICOS than the wild-type ICOS-L, and in particular, the Y8 variant showed higher binding affinity than the conventional A184 (FIG. 8).

### Example 8. Search for critical mutations of ICOS-L with enhanced binding affinity for ICOS

### 8-1. Preparation of mutation recovery variants

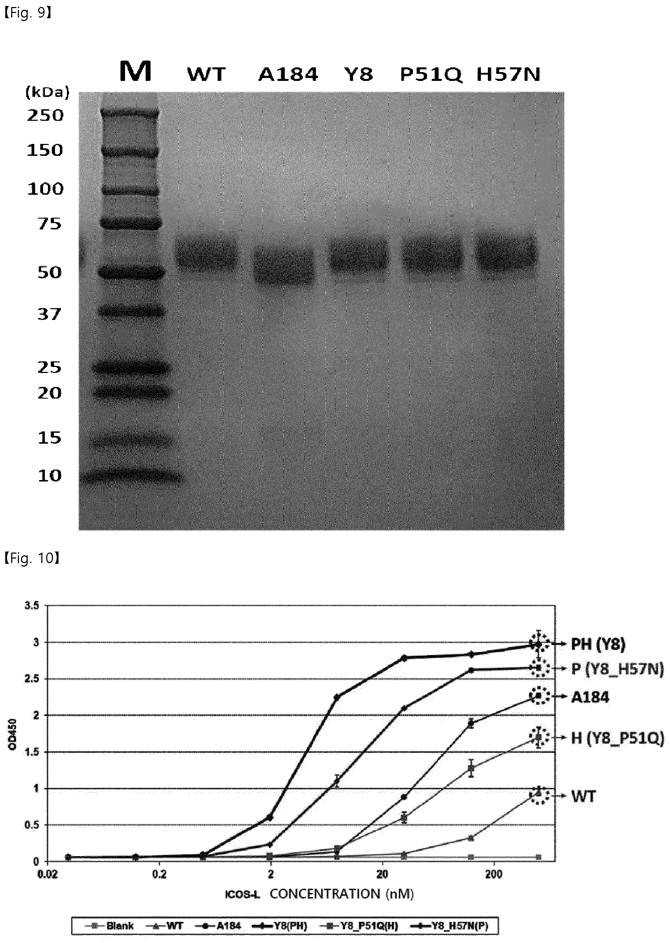

To search for a critical mutation in the Y8 variant that had enhanced binding affinity compared to a control A184, Y8_P51Q (P51 of the Y8 variant was substituted with Q) and Y8_H57N (H57 of the Y8 variant was substituted with N), variants in which the two mutations in Y8 were each substituted with an amino acid sequence of the wild-type ICOS-L were prepared. To construct animal cell expression vectors for the Y8_P51Q and Y8_H57N variants, the genome was amplified by Quikchange PCR technique using primers and Pfu turbo polymerase (Agilent) designing the prepared pMAZ-ICOS-L_Y8 plasmid. The amplified gene was transformed into Jude1 and a sequence was confirmed. The constructed ICOS-L variant expression vectors (pMAZ-ICOS-L_WT, pMAZ-ICOS-L_A184, pMAZ-ICOS-L_Y8, pMAZ-ICOS-L_Y8_P51Q, and pMAZ-ICOS-L_Y8_H57N) were transfected into Expi293F animal cells, cultured for 7 days, and then centrifuged to separate only the supernatant. Thereafter, the cells were equilibrated using 25x PBS and filtered through a 0.2 µm filter (Thermo Fisher Scientific) using a Bottle top filter. The filtered culture medium was added with 0.5 ml of Ni-NTA resin, stirred at 4°C for 16 hours, and then the resin was recovered using a column, washed with a PBS solution containing 20 column volume (CV) of 10 mM imidazole (Sigma), and then washed once more with a PBS solution containing 20 CV of 20 mM imidazole. Thereafter, the culture medium was eluted with a PBS solution containing 250 mM imidazole, and then the buffer was changed to 1X PBS (pH 7.4) using centrifugal filter units 10K (Merck Millipore). As a result, four purified variants of each 1 mg or more were obtained. Size exclusion chromatography (NGC Chromatography, Biorad) was performed to increase the purity of the purified ICOS-L variants. Aggregated proteins were removed using a Superdex 200 10/300 GL column (Cytiva), and only fractions 21 and 22 were recovered. Thereafter, it was confirmed through SDS-PAGE that the purity of the five ICOS-L variants was increased (FIG. 9).

### 8-2. Search of critical mutations in Y8 variant

ELISA was performed to search for critical mutations in the Y8 variant. Specifically, 50 µl of ICOS-Fcs diluted to 4 µg/ml in 0.05 M Na₂CO₃ (pH 9.6) was dispensed into a Flat Bottom Polystyrene High Bind 96-well microplate (Costar), immobilized at 4°C for 16 hours, and then blocked with 100 µl of 0.5% BSA (Gibco) (in PBS, pH 7.4) for 2 hours at room temperature. After washing four times with 180 µl of 0.05% PBST (pH 7.4), 50 µl of ICOS-L variants serially diluted with 0.5% BSA (in PBS pH 7.4) and prepared and purified in Example 8-1 were dispensed into each well and reacted at room temperature for 1 hour. After washing four times, 50 µl of an anti-His-HRP conjugate was added and reacted at room temperature for 1 hour, and then washed four times. Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction, and then analyzed using an Epoch microplate spectrophotometer (BioTek).

As a result, P51 mutation appeared to have a greater effect on enhancing binding affinity than H57 mutation of Y8, and the binding affinity was found to be the best when the P51 and H57 mutations existed together (FIG. 10).

### Example 9. Comparison of binding affinity of ICOS-L variants containing critical mutations for ICOS

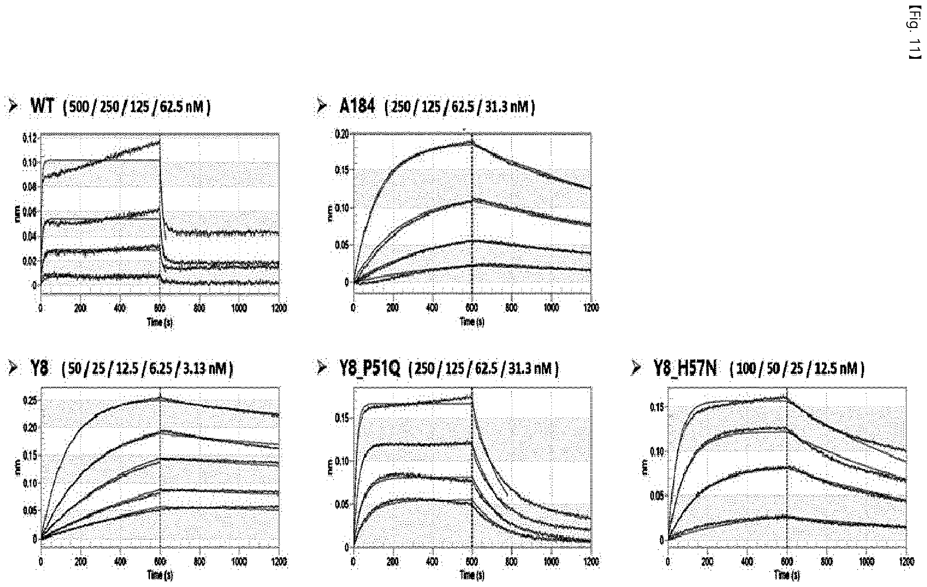

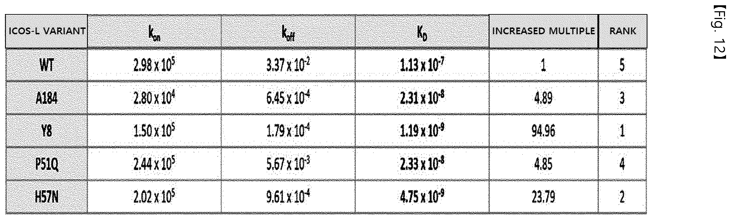

The binding affinity of wild-type ICOS-L, conventional A184 variant, and ICOS-L variants Y8, Y8_P51Q, and Y8_H57N for ICOS was measured and compared using a Bio-layer interferometry assay (Octet R8, Satorius). Specifically, 5 µg/ml of ICOS-Fc was immobilized for 1 minute on a Protein A biosensor (Satorius) that was equilibrated with 1X PBS (pH 7.4) for 1 minute, and then after establishing a baseline for 1 minute with 1X PBS (pH 7.4), ICOS-L variants at concentrations of 3.13 nM to 500 nM were associated for 10 minutes, and dissociated by flowing in 1X PBS (pH 7.4) for 10 minutes. In this way, concentration-specific sensor grams of respective variants were analyzed (FIG. 11), and final dissociation equilibrium constant (KD) values were analyzed (FIG. 12).

### Example 10. Preparation of ICOS monomers for measuring binding affinity of ICOS-L variants

### 10-1. ICOS monocloning

To measure the ICOS binding affinity of various forms of ICOS-L variants, the gene was amplified using primers designed with DNA encoding an amino acid sequence E21-H129 of the extracellular domain of ICOS, which eliminated the possibility of ICOS dimer formation, and Vent Polymerase (New England Biolab). The amplified gene was treated with a restriction enzyme using BssHII and XbaI (New England Biolab), and the restriction enzyme-treated ICOS (E21-H129) gene was ligated into a pMAZ vector, which was a vector for animal cells treated with the same restriction enzyme. The ligated plasmid was transformed into Jude1 E. coli to obtain monoclones and through sequencing, it was confirmed that the ICOS (E21-H129) was successfully inserted into the pMAZ vector.

### 10-2. Expression and purification of ICOS monomer protein

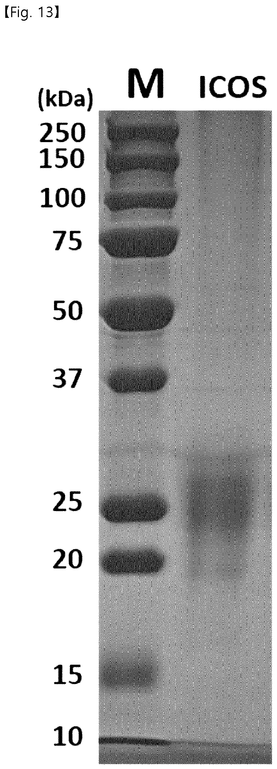

The constructed ICOS expression vector was transfected into Expi293F animal cells and cultured in a CO₂ shaking incubator at 37°C, 125 rpm, and 8% CO₂ for 7 days, and then centrifuged to separate only the supernatant. Thereafter, the culture medium was equilibrated using 25x PBS and filtered using a 0.2 µm bottle top filter (Merck Millipore). The filtered culture medium was added with 0.5 ml of Ni-NTA resin, stirred at room temperature for 1 hour to recover the resin, and washed with 10 ml of a PBS solution containing 10 mM imidazole (Sigma) and washed once more with 10 ml of a PBS solution containing 20 mM imidazole. The culture medium was eluted with a PBS solution containing 250 mM imidazole, and then the buffer was changed to 1X PBS (pH 7.4) using centrifugal filter units 10K (Merck Millipore). Thereafter, the expressed and purified ICOS monomer protein was confirmed by SDS-PAGE gel (FIG. 13).

### Example 11. Preparation of ICOS-L variants fused to antibody Fc domain

### 11-1. Cloning of ICOS-L variants fused to antibody Fc domain

To evaluate the T cell activation ability of the discovered ICOS-L variants, the ICOS-L variants were fused to a silent Fc variant LALAPG (L234A/L235A/P329G) of a human IgG1 antibody. The ICOS-L and Fc (LALAPG) genes were amplified using primers designed to fuse the Fc domain of the human IgG1 antibody to the C-terminal portion of wild-type ICOS-L, conventional A184 variant, and ICOS-L variants Y8 and Y8_H57N of the present disclosure and Vent Polymerase (New England Biolab). The amplified genes were subjected to assembly PCR and then inserted into pMAZ vectors (pMAZ-ICOS-L_WT-Fc, pMAZ-ICOS-L_A184-Fc, pMAZ-ICOS-L_Y8-Fc, and pMAZ-ICOS-L_Y8_H57N-Fc) using 2× Gibson assembly master mix (New England Biolab). The gene sample that completed the reaction was transformed into Jude1 E. coli, and then monoclones were obtained and subjected to sequencing, and as a result, it was confirmed that four ICOS-L variants fused to Fc (LALAPG) were successfully inserted into the pMAZ vector.

### 11-2. Expression and purification of ICOS-L variants fused to antibody Fc domain

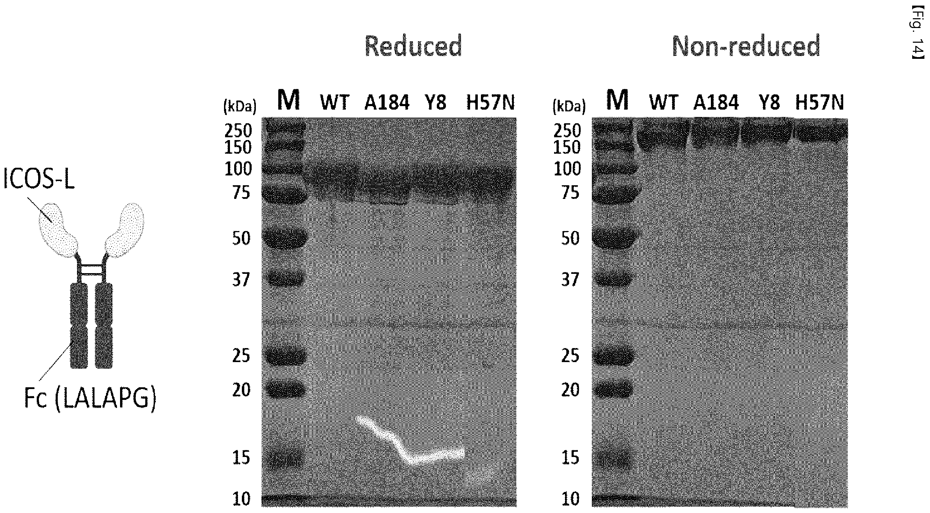

100 ml of Expi293F cells were subcultured at a density of 2 × 10⁶ cells/ml, and one day later, the Fc-fused ICOS-L variant expression vectors (pMAZ-ICOS-L_WT-Fc, pMAZ-ICOS-L_A184-Fc, pMAZ-ICOS-L_Y8-Fc and pMAZ-ICOS-L_Y8_H57N-Fc) prepared in Example 11-1 were mixed with PEI (Polyehylenimine, Polyscience, 23966) in a ratio of 1:4 in 10 ml of a Freestyle 293 expression culture medium (Gibco. 12338-018), left at room temperature for 20 minutes, and then transfected into the Expi293F animal cells. The cells were cultured in a CO₂ shaking incubator at 37°C, 125 rpm, and 8% CO₂ for 7 days, centrifuged, and only the supernatant was collected, and then the culture medium was equilibrated with 25× PBS and filtered through a 0.2 µm filter (Merck Millipore) using a bottle top filter. The filtered culture medium was added with 0.5 ml of Protein A resin, passed through a column, and the resin was recovered, and then washed with 10 ml of PBS. The washed resin was eluted with 100 mM of glycine pH 2.7 buffer and then neutralized using 1 M Tris-HCl pH 8.0 to obtain a purified ICOS-L variant fusion protein, and the buffer was replaced with 1x PBS using Centrifugal filter units 10K (Merck Millipore). ICOS-L variants fused to the expressed and purified antibody Fc domain (LALAPG) were identified by SDS-PAGE gel (FIG. 14).

### 11-3. Verification of binding affinity of ICOS-L variants fused to antibody Fc domain for ICOS

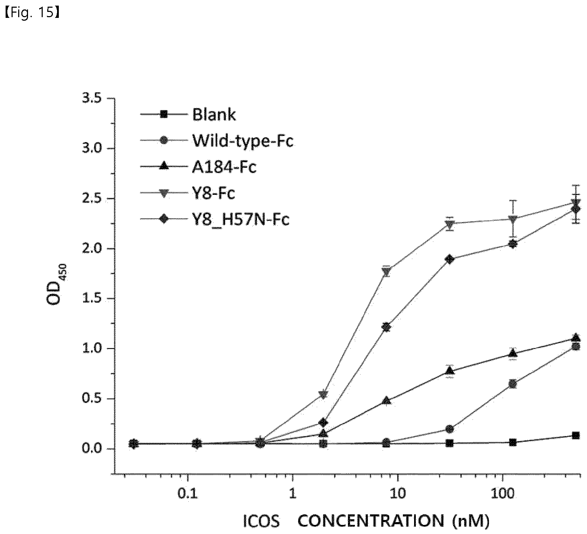

To analyze the ICOS binding affinity of ICOS-L variants fused to the antibody Fc domain (LALAPG) purified in Example 11-2 above, ELISA assay was performed. Specifically, 50 µl of ICOS-L-Fcs diluted to 4 µg/ml in 0.05 M Na₂CO₃ (pH 9.6) was dispensed into a Flat Bottom Polystyrene High Bind 96-well microplate (Costar), immobilized at 4°C for 16 hours, and then blocked with PBS (pH 7.4) containing 100 µl of 0.5% bovine serum albumin (BSA) (Gibco) for 2 hours at room temperature. After washing four times with 180 µl of 1× PBS (pH 7.4) (PBST) containing 0.05% Tween20, 50 µl of wild-type ICOS serially diluted with PBS (pH 7.4) containing 0.5% BSA was dispensed into each well and reacted at room temperature for 1 hour. After washing four times, 50 µl of an anti-His-HRP conjugate was added and reacted at room temperature for 1 hour, and then washed four times. Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction, and then analyzed using an Epoch microplate spectrophotometer (BioTek). As a result, it was confirmed that the ICOS-L variants fused to the antibody Fc domain (LALAPG) bound to wild-type ICOS at the same order as before fusion of Fc (FIG. 15).

### Example 12. Confirmation of T cell activation of ICOS-L variants fused to antibody Fc domain

### 12-1. Verification of T cell activity through T cell differentiation analysis

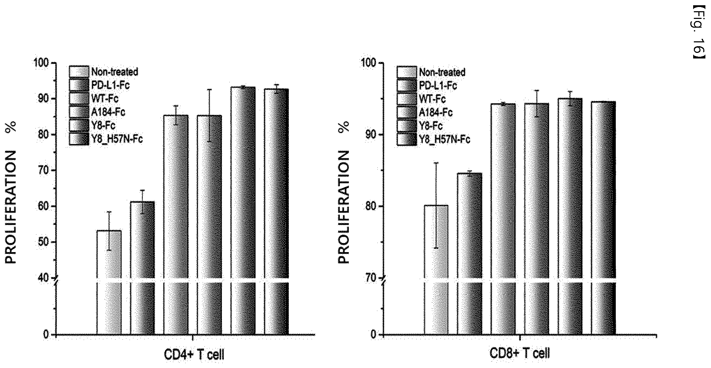

To confirm the T cell activation ability of ICOS-L variants fused to the antibody Fc domain through co-stimulation signaling, the degree of T cell differentiation was confirmed. Specifically, 1.5 µg/ml anti-CD3 antibody (Biolegend) and 50 µl of each of 50 nM Fc-fused ICOS-L variants were dispensed into a Flat Bottom Cell Culture Plate (SPL), immobilized at 4°C for 20 hours, and then washed three times with 200 µl of PBS (pH 7.4). T cells were isolated from human Peripheral Blood Mononuclear Cells (PBMCs) using a Pan T Cell Isolation Kit (Miltenyi Biotec), stained with Carboxyfluorescein diacetate succinimidyl ester (CFSE) (Invitrogen), resuspended in an AIM-V medium (Gibco), and then dispensed at 5 × 10⁴ cells in a protein-immobilized plate. The cells were cultured for 3 days in a CO₂ cell incubator at 37°C and 5% CO₂, and then the cultured T cell culture medium was centrifuged at 400 × g for 5 minutes, resuspended in 100 µl of PBS, and the cells were recovered using a V-bottom cell culture plate (Corning). The recovered cells were centrifuged and resuspended in 50 µl of PBS, and added with 50 µl of anti-CD4-APC probe or anti-CD8-APC probe (200:1), and incubated for 20 minutes at room temperature to label the cells with the fluorescent probe. Thereafter, the cells were washed with 100 µl of PBS, centrifuged (400 × g, 5 min), and then resuspended in 200 µl of PBS to prepare the samples, and a decrease in CFSE fluorescence signal value of the prepared samples was measured using a FACSLyric (BD Biosciences) device. As a result, it was confirmed that T cell activation was induced and T cell differentiation occurred by ICOS-L variants fused to the antibody Fc domain (FIG. 16). Through this, it was confirmed that the ICOS-L variants of the present disclosure with enhanced binding affinity for ICOS successfully induced differentiation of CD4+ T cells and CD8+ T cells.

### 12-2. Verification of T cell activity through cytokine secretion analysis

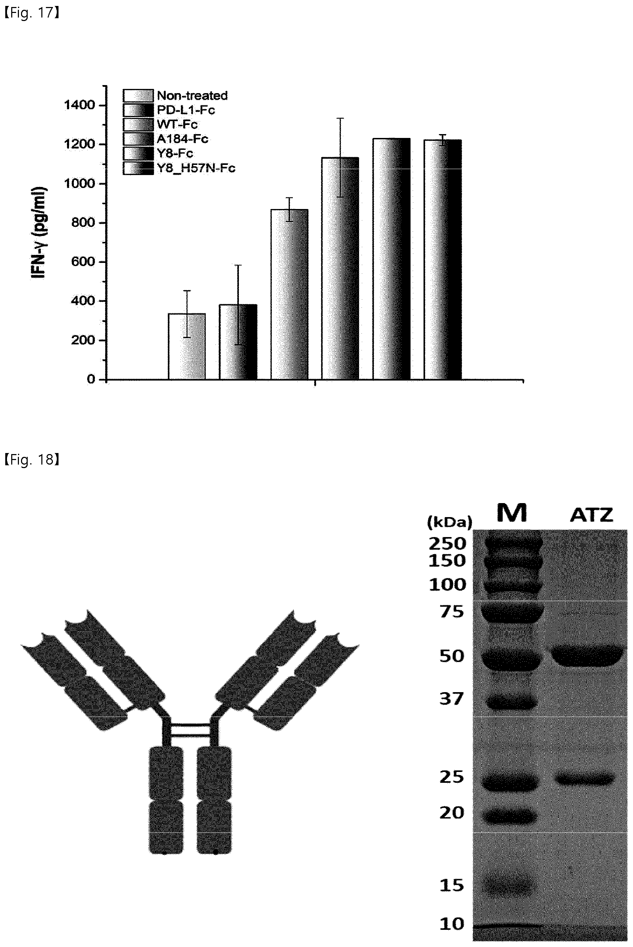

To confirm the T cell activation ability of ICOS-L variants fused to the antibody Fc domain through co-stimulation signaling, the degree of cytokine (interferon gamma) secretion was confirmed. Specifically, T cells were isolated as in Example 12-1 above, stained with CFSE (Carboxyfluorescein diacetate succinimidyl ester) (Invitrogen), resuspended in an AIM-V medium (Gibco), and then dispensed at 5 × 10⁴ cells in a protein-immobilized plate. The culture medium cultured for 3 days in a CO₂ cell incubator at 37°C and 5% CO₂ was centrifuged at 400 × g for 5 minutes, and the supernatant was obtained to verify the T cell activation ability of ICOS-L variants fused to the antibody Fc domain using a DuoSet Human IFN-γ ELISA Kit (R&D Systems). As a result, it was shown that the variants fused to the antibody Fc domain also significantly enhanced cytokine secretion (FIG. 17).

### Example 13. Preparation of ICOS-L variants fused to antibody

### 13-1. Preparation of anti-PD-L1 antibody (Atezolizumab)

### 13-1-1. Cloning of Atezolizumab expression vector

As an example of the application of ICOS-L variants, to determine whether the efficacy of the antibody may be maximized by fusing ICOS-L variants to the antibody, Atezolizumab, an anti-PD-L1 antibody was prepared. To improve purification yield and protein stability compared to conventional antibodies, the effector function of the antibody was reduced by introducing the LALAPG mutation, instead of conventional IgG1 N297A mutation, into the Fc domain of Atezolizumab. Specifically, assembly PCR was performed by amplifying a variable region gene of the heavy chain of Atezolizumab and a constant region gene of the heavy chain with the LALAPG mutation using designed primers and Vent Polymerase (New England Biolab), respectively, and the obtained genes were treated with BssHII and XbaI (New England Biolab). In addition, the variable region gene of the light chain of Atezolizumab was treated with the same restriction enzyme by performing assembly PCR with the constant region gene of the light chain. The heavy and light chain genes treated with the restriction enzyme were ligated into the pMAZ vector, a vector for animal cells treated with the same restriction enzyme, the ligated plasmid was transformed into Jude1 E. coli to obtain monoclones, and through sequencing, it was confirmed that the heavy and light chain genes of Atezolizumab, from which the effector function was removed, were successfully inserted into the pMAZ vector.

### 13-1-2. Expression and purification of Atezolizumab

The heavy and light chain genes of Atezolizumab prepared in Example 13-1-1 above were mixed in a 1:1 ratio, mixed with PEI (Polyehylenimine, Polyscience, 23966) in a 1:4 ratio, left at room temperature for 20 minutes, and then transfected into Expi293F animal cells. The cells were cultured for 7 days in a CO₂ shaking incubator at 37°C, 125 rpm, and 8% CO₂, and then centrifuged to separate only the supernatant. Thereafter, the culture medium was equilibrated with 25x PBS and filtered through a 0.2 µm filter (Merck Millipore) using a bottle top filter. The filtered culture medium was added with 1 ml of Protein A resin, passed through a column, and the resin was recovered, and then washed with 10 ml of PBS. The washed resin was eluted with 100 mM of glycine pH 2.7 buffer and then neutralized using 1 M Tris-HCl (pH 8.0) to obtain purified Atezolizumab, and the buffer was replaced with 1x PBS using Centrifugal filter units 30K (Merck Millipore). Thereafter, the expressed and purified Atezolizumab antibody (ATZ) was identified by SDS-PAGE gel (FIG. 18).

### 13-2. Preparation of ICOS-L variants fused to anti-PD-L1 antibody

### 13-2-1. Cloning of ICOS-L variants fused to Atezolizumab

The ICOS-L variants of the present disclosure were cloned to be fused to Atezolizumab (IgG1 Fc-LALAPG), an anti-PD-L1 antibody. Specifically, ICOS-L, and heavy chain and light chain genes of Atezolizumab were amplified using primers designed to fuse wild-type ICOS-L, conventional ICOS-L A184 variant, and ICOS-L variants Y8 and Y8_H57N of the present disclosure to the C-terminal of the heavy chain or the C-terminal of the light chain of Atezolizumab, respectively, and Vent Polymerase (New England Biolab). After performing assembly PCR, the amplified genes were inserted into the pMAZ vector using 2× Gibson assembly master mix (New England Biolab). The gene samples that completed the reaction were transformed into Jude1 E. coli, monoclones were obtained and the sequencing was analyzed, and as a result, it was confirmed that the ICOS-L variants fused to the C-terminal of the heavy or the C-terminal of the light chain of Atezolizumab were successfully inserted into the pMAZ vector.

### 13-2-2. Expression and purification of ICOS-L variants fused to Atezolizumab

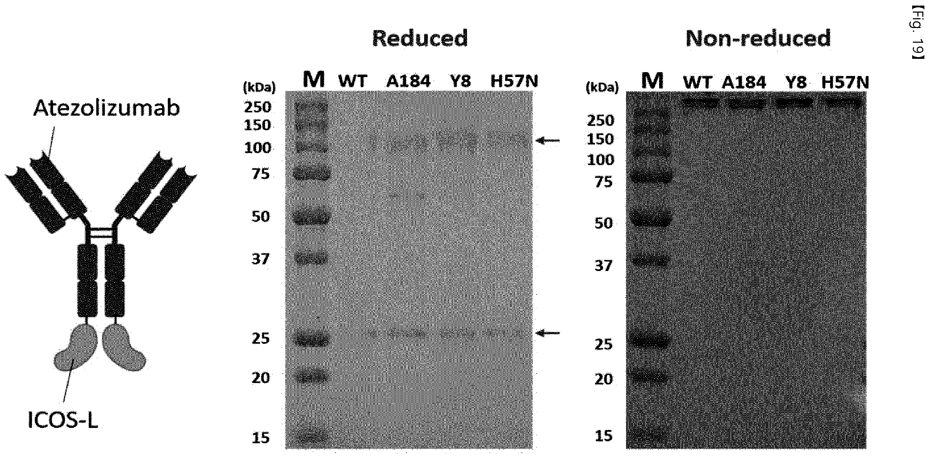

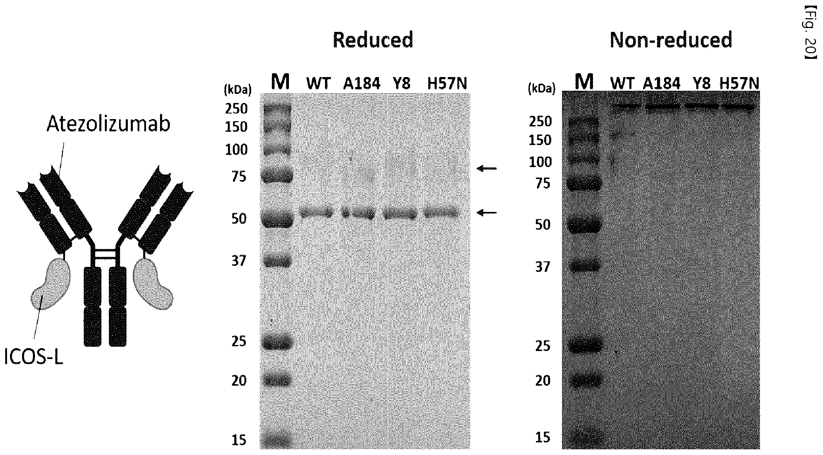

100 ml of Expi293F cells were subcultured at a density of 2 × 10⁶ cells/ml, and one day later, an ICOS-L variant expression vector (pMAZ-Atezolizumab-IgH-ICOS-L_WT, pMAZ-Atezolizumab-IgH-ICOS-L_A184, pMAZ-Atezolizumab-IgH-ICOS-L_Y8 or pMAZ-Atezolizumab-IgH-ICOS-L_Y8_H57N) (+ pMAZ-Atezolizumab-IgL) fused to the heavy chain of Atezolizumab prepared in Example 13-2-1 and an ICOS-L variant expression vector (pMAZ-Atezolizumab-IgL-ICOS-L_WT, pMAZ-Atezolizumab-IgL-ICOS-L_A184, pMAZ-Atezolizumab-IgL-ICOS-L_Y8 or pMAZ-Atezolizumab-IgL-ICOS-L_Y8_H57N) (+ pMAZ-Atezolizumab-IgH) fused to the light chain of Atezolizumab were mixed with PEI (Polyehylenimine, Polyscience, 23966) in a ratio of 1:4 in 10 ml of a Freestyle 293 expression culture medium (Gibco. 12338-018), left at room temperature for 20 minutes, and then transfected into the Expi293F animal cells. The cells were incubated for 7 days under conditions of 37°C, 125 rpm, and 8% CO₂ in a CO₂ shaking incubator, and then centrifuged to collect only a supernatant. Thereafter, the culture medium was equilibrated with 25x PBS and filtered through a 0.2 µm filter (Merck Millipore) using a bottle top filter. The filtered culture medium was added with 0.5 ml of Protein A resin, passed through a column, and the resin was recovered, and then washed with 10 ml of PBS. The washed resin was eluted with 100 mM of glycine (pH 2.7) buffer and then neutralized using 1 M Tris-HCl (pH 8.0) to obtain ICOS-L variants fused to the purified heavy or light chain of Atezolizumab, and the buffer was replaced with 1x PBS using Centrifugal filter units 10K (Merck Millipore). In addition, the expressed and purified ICOS-L variants fused to the heavy or light chain of Atezolizumab were identified by SDS-PAGE gel (FIGS. 19 and 20).

### 13-3. Verification of binding affinity of ICOS-L variants fused to anti-PD-L1 antibody for ICOS

To analyze the ICOS binding affinity of ICOS-L variants fused to the heavy chain or light chain of Atezolizumab purified in Example 13-2 above, ELISA assay was performed. Specifically, 50 µl of 40 nM PD-L1-Fc in 0.05 M Na₂CO₃ (pH 9.6) was dispensed into a Flat Bottom Polystyrene High Bind 96-well microplate (Costar), immobilized at 4°C for 16 hours, and then blocked with 100 µl of PBS (pH 7.4) containing 0.5% bovine serum albumin (BSA) (Gibco) for 2 hours at room temperature. After washing four times with 180 µl of PBST (pH 7.4), 200 nM of ICOS-L variants fused to the heavy or light chain of Atezolizumab diluted in PBS (pH 7.4) containing 0.5% BSA were reacted for 1 hour at room temperature. After washing four times, 50 µl of wild-type ICOS serially diluted with 0.5% BSA (in PBS pH 7.4) was dispensed into each well and reacted at room temperature for 1 hour. After washing four times, 50 µl of an anti-His-HRP conjugate was added and reacted at room temperature for 1 hour, and then washed four times. Thereafter, 50 µl of a 1-Step Ultra TMB-ELISA substrate solution (Thermo Fisher Scientific, 34028) was added to develop color, and then added with 50 µl each of 2 M H₂SO₄ to terminate the reaction, and then analyzed using an Epoch microplate spectrophotometer (BioTek). As a result, it was shown that ICOS-L variants (ATZ(H)-A184, ATZ(H)-Y8, and ATZ(H)-Y8_H57N) fused to the heavy chain of Atezolizumab and ICOS-L variants (ATZ(L)-A184, ATZ(L)-Y8 and ATZ(L)-Y8_H57N) fused to the light chain bound to wild-type ICOS at the same order as before fusion to Atezolizumab (FIGS. 21 and 22).

### Example 14. Verification of T cell activation ability of ICOS-L variants fused to antibody

### 14-1. Verification of T cell activity through T cell differentiation analysis

To verify the T cell activation ability of ICOS-L variants fused to the heavy chain or light chain of Atezolizumab through co-stimulation signaling, the degree of T cell differentiation was confirmed as in Example 12-1 above. As a result, it was confirmed that the T cell activation was induced and T cell differentiation occurred by the ICOS-L variants fused to the heavy chain or light chain of Atezolizumab (FIGS. 23 and 24). Through this, it was confirmed that the ICOS-L variants still successfully induced differentiation of CD4+ T cells and CD8+ T cells even when fused to Atezolizumab.

### 14-2. Verification of T cell activity through cytokine secretion analysis

To verify the T cell activation ability of ICOS-L variants fused to the heavy chain or light chain of Atezolizumab through co-stimulation signaling, the degree of cytokine (interferon gamma) secretion was confirmed as in Example 12-2 above. As a result, cytokine secretion from T cells treated with Atezolizumab fused to ICOS-L was significantly increased compared to the Atezolizumab antibody (FIGS. 25 and 26). Through this, it was confirmed that ICOS-L maximized the efficacy of Atezolizumab by successfully inducing the T cell activation.

## Claims

1. An ICOS-L variant in which any one or more amino acids selected from the group consisting of amino acids at positions 4, 51, 52, 54, 57, 74, 130, and 234 in an amino acid sequence of wild-type Inducible Co-Stimulator-ligand (ICOS-L) are substituted with sequences different from amino acids of the wild type.

2. The ICOS-L variant of claim 1, wherein the amino acids of the wild-type ICOS-L includes an amino acid sequence of SEQ ID NO: 1.

3. The ICOS-L variant of claim 1, comprising: any one or more amino acid substitutions selected from the group consisting of E4D, Q51P, Q51H, N52S, S54N, N57H, L74R, S130C and K234R.

4. The ICOS-L variant of claim 1, comprising: amino acid substitutions of Q51P and N57H.

5. The ICOS-L variant of claim 1, comprising: amino acid substitutions of Q51H, S54N and S130C.

6. The ICOS-L variant of claim 1, comprising: amino acid substitutions of N52S and K234R.

7. The ICOS-L variant of claim 1, comprising: amino acid substitutions of E4D and Q51P.

8. The ICOS-L variant of claim 1, comprising: amino acid substitutions of Q51P and L74R.

9. The ICOS-L variant of claim 1, further comprising: a transmembrane domain.

10. The ICOS-L variant of claim 9, further comprising:
a cytoplasmic signaling domain linked to the transmembrane domain.

11. An immunomodulatory protein comprising the ICOS-L variant of claim 1 and an Fc domain of immunoglobulin or a variant thereof.

12. The immunomodulatory protein of claim 11, wherein the Fc domain variant comprises one or more amino acid substitutions in an Fc domain of wild-type human IgG1.

13. The immunomodulatory protein of claim 11, wherein the Fc domain variant is an Fc domain variant that exhibits a reduced effector function compared to the Fc of wild-type human IgG1.

14. The immunomodulatory protein of claim 11, wherein in the Fc domain variant, amino acids at position 234, 235 or 329, numbered according to the Kabat numbering system in the Fc domain of wild-type human IgG1 are substituted with sequences different from amino acids of the wild-type.

15. The immunomodulatory protein of claim 11, wherein the Fc domain variant comprises amino acid substitutions of L234A, L235A and P329G.

16. A conjugate comprising the ICOS-L variant of claim 1 or the immunomodulatory protein of claim 11, and a targeting moiety.

17. The conjugate of claim 16, wherein the targeting moiety specifically binds to a molecule on the surface of an immune cell.

18. The conjugate of claim 17, wherein the immune cell is an antigen presenting cell (APC) or a lymphocyte.

19. The conjugate of claim 16, wherein the targeting moiety is a tumor-localizing moiety that binds to a molecule on the tumor surface.

20. The conjugate of claim 16, wherein the targeting moiety is an antibody or an immunologically active fragment thereof.

21. The conjugate of claim 20, wherein the antibody is cetuximab, panitumumab, zalutumumab, nimotuzumab, trastuzumab, ado-trastuzumab, emtacin, tositumomab (Bexxar), rituximab (Rituxan, MabThera), ibritumomab tiuxetan (Zevalin), daclizumab (Zenapax), gemtuzumab (Mylotarg), alemtuzumab, CEA-scan Fab fragment, OC125 monoclonal antibody, ab75705, B72.3, bevacizumab (Avastin), afatinib, axitinib, bosutinib, cabozatinib, ceritinib, crizotinib, dabrafenib, dasatinib, dinutuximab, erlotinib, everolimus, ibrutinib, imatinib, lapatinib, lenvatinib, nilotinib, olaparib, olaratumab, palbociclib, Pazopanib, pertuzumab, ramucirumab, regorafenib, ruxolitinib, sorafenib, sunitinib, temsirolimus, trametinib, vandetanib, vemurafenib, vismodegib, basiliximab, ipilimumab, nivolumab, pembrolizumab, MPDL3280A, pidilizumab (CT-011), AMP-224, MSB001078C, or MEDI4736, BMS-935559, LY3300054, Atezolizumab, avelumab, or durvalumab.

22. A T-cell activator comprising the ICOS-L variant of claim 1, the immunomodulatory protein of claim 11, or the conjugate of claim 16.

23. The T-cell activator of claim 22, wherein the T cell is a cytotoxic T cell or a chimeric antigen receptor (CAR)-T cell.

24. A polynucleotide comprising a nucleic acid encoding the ICOS-L variant of claim 9 and one or more nucleic acids encoding one or more chains of a recombinant antigen receptor.

25. The polynucleotide of claim 24, wherein the recombinant antigen receptor is a chimeric antigen receptor (CAR) or an engineered T cell receptor (TCR).

26. An engineering cell comprising the ICOS-L variant of claim 1, the immunomodulatory protein of claim 11, or the conjugate of claim 16.

27. The engineering cell of claim 26, further comprising:
a chimeric antigen receptor (CAR) or an engineered T cell receptor (TCR).

28. An anticancer adjuvant comprising the ICOS-L variant of claim 1, the immunomodulatory protein of claim 11, the conjugate of claim 16, or the engineering cell of claim 26.

29. The anticancer adjuvant of claim 28, wherein the anticancer adjuvant is a cancer immunotherapy adjuvant that enhances an anticancer effect of immune checkpoint inhibitors.

30. The anticancer adjuvant of claim 28, wherein the anticancer adjuvant is co-administered simultaneously, separately or sequentially with the immune checkpoint inhibitors.

31. The anticancer adjuvant of claim 29, wherein the immune checkpoint inhibitor is an anti-PD-1 antibody, an anti-PD-L1 antibody, an anti-CTLA-4 antibody, or a variant thereof.

32. A pharmaceutical composition for preventing or treating cancer, comprising the ICOS-L variant of claim 1, the immunomodulatory protein of claim 11, the conjugate of claim 16, or the engineering cell of claim 26 as an active ingredient.

33. The pharmaceutical composition for preventing or treating cancer of claim 32, wherein the cancer is any one or more selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

34. A composition for diagnosing cancer comprising the ICOS-L variant of claim 1, the immunomodulatory protein of claim 11, the conjugate of claim 16, or the engineering cell of claim 26.

35. The composition for diagnosing cancer of claim 34, further comprising:
one selected from the group consisting of a chromogenic enzyme, a radioisotope, a chromophore, a luminescent material, and a fluorescent material.

36. A method for providing information for cancer diagnosis comprising:
a) contacting a biological sample isolated from a subject with the composition of claim 34;
b) confirming the expression of ICOS in the sample; and
c) comparing the expression of ICOS in a normal control sample.

37. A method for predicting therapeutic response or diagnosing prognosis for an immune checkpoint inhibitor, comprising confirming the expression level of ICOS using the ICOS-L variant of claim 1, the immunomodulatory protein of claim 11, the conjugate of claim 16, or the engineering cell of claim 26 in a biological sample isolated from a subject treated with the immune checkpoint inhibitor.

38. A use of the ICOS-L variant of claim 1, the immunomodulatory protein of claim 11, the conjugate of claim 16, or the engineering cell of claim 26 for preventing or treating cancer.

39. A method for treating cancer comprising administering the ICOS-L variant of claim 1, the immunomodulatory protein of claim 11, the conjugate of claim 16, or the engineering cell of claim 26 in a pharmaceutically effective amount to a subject suffering with cancer.
